# EUROPEAN PATENT SPECIFICATION

(11) **EP 1 451 219 B1**
(45) Date of publication and mention of the grant of the patent: **08.08.2007**
(21) Application number: 02787902.2
(22) Date of filing: 03.12.2002
(51) Int. Cl.: C07K 14/47, G01N 33/53, C12N 15/12, C07K 16/18, C12N 5/10, C12N 15/63, A61K 38/16

(54) **NUCLEAR PROTEIN "SHOCA" - A COMPONENT OF THE WNT SIGNALLING PATHWAY**
NUKLEARES PROTEIN "SHOCA" - EINE KOMPONENTE DES WNT SIGNALTRANSDUKTIONSWEGES
PROTEINE NUCLEAIRE "SHOCA" - UNE COMPOSANTE DE LA VOIE DE SIGNALISATION WNT

(30) Priority: 06.12.2001 GB 0129278; 06.12.2001 US 336176 P
(43) Date of publication of application: 01.09.2004
(73) Proprietor: The University Children's Hospital of Both Cantons of Basel, 4000 Basel (CH)
(72) Inventor: HOLLANDER, Georg Pediatric Immunology, Mattenstrasse 28 CH-4058 Basel (CH)
(74) Representative: Roques, Sarah Elizabeth
(86) International application number: PCT/EP2002/013675
(87) International publication number: WO 2003/048200

(56) References cited:
- DATABASE EMBL [Online] 30 November 2000 (2000-11-30) retrieved from EBI Database accession no. BB599521 XP002240709
- DATABASE EMBL [Online] 14 January 2000 (2000-01-14) retrieved from EBI Database accession no. AC021028 XP002240710
- PEIFER MARK ET AL: "Wnt signaling in oncogenesis and embryogenesis-a look outside the nucleus." SCIENCE (WASHINGTON D C), vol. 287, no. 5458, 3 March 2000 (2000-03-03), pages 1606-1609, XP002240707 ISSN: 0036-8075 cited in the application
- POLAKIS PAUL: "Wnt signaling and cancer." GENES & DEVELOPMENT, vol. 14, no. 15, 1 August 2000 (2000-08-01), pages 1837-1851, XP002240708 ISSN: 0890-9369 cited in the application

### SEQUENCE LISTING

<110> The University Children's Hospital of both Cantons of Basel
<120> NUCLEAR PROTEIN
<130> N84006
<160> 32
<170> PatentIn version 3.0
<210> 1
   <211> 1296
   <212> DNA
   <213> Mus musculus
<220
   <221> CDS
   <222> (1)..(1296)
<400>: 1
   atg ctg cag cag atc ctg cag gac atg tac atc gac ccg gag ctg ttg      48
   Met Leu Gln Gln Ile Leu Gln Asp Met Tyr Ile Asp Pro Glu Leu Leu
   1               5                   10                  15
   gcc gag ctc agc gat gtg cag aag cac atc ctc ttc tat aag atg cga      96
   Ala Glu Leu Ser Asp Val Gln Lys His Ile Leu Phe Tyr Lys Met Arg
               20                  25                  30
   gag gag cag ctg cgg cga tgg agg gaa cgg gag gcg tgg gat gcc ctg      144
   Glu Glu Gln Leu Arg Arg Trp Arg Glu Arg Glu Ala Trp Asp Ala Leu
           35                  40                  45
   gct cag gca gag ggc ctg agg ccc gca aag gtc aag aga gcg agc aac      192
   Ala Gln Ala Glu Gly Leu Arg Pro Ala Lys Val Lys Arg Ala Ser Asn
       50                  55                  60
   aag cat ctc cag tgg ctt ctg gga gca gat ggt gag gtc tgg gtg tgg      240
   Lys His Leu Gln Trp Leu Leu Gly Ala Asp Gly Glu Val Trp Val Trp
   65                  70                  75                  80
   gtc atg ggc gaa ggc cct gga gac aag ccc tac gaa gag ata tct gaa      288
   Val Met Gly Glu Gly Pro Gly Asp Lys Pro Tyr Glu Glu Ile Ser Glu
                   85                  90                  95
   gaa ctg att gca gag agg gcc cgg ctg caa gca cag aaa gag gct gaa      336
   Glu Leu Ile Ala Glu Arg Ala Arg Leu Gln Ala Gln Lys Glu Ala Glu
               100                 105                 110
   gag ctc tgg aga cag aag gaa gca gag atc acc aag aag ttc cgg gat      384
   Glu Leu Trp Arg Gln Lys Glu Ala Glu Ile Thr Lys Lys Phe Arg Asp
           115                 120                 125
   gcc ttg gcc aat gag aaa gct cgg att ctg gca gag aag tgg aaa gtg      432
   Ala Leu Ala Asn Glu Lys Ala Arg Ile Leu Ala Glu Lys Trp Lys Val
       130                 135                 140
   gag atg gag gac cgc aag gct gct aaa att ttg gag gaa cgt ata cac      480
   Glu Met Glu Asp Arg Lys Ala Ala Lys Ile Leu Glu Glu Arg Ile His
   145                 150                 155                 160
   gag gaa ttc aag agg aaa gag gaa gaa gag agg cgg cga ggg gaa gaa      528
   Glu Glu Phe Lys Arg Lys Glu Glu Glu Glu Arg Arg Arg Gly Glu Glu
                   165                 170                 175
   cag att cgg ctc caa gag gag cag agg gca aag gaa ctc tac tgg act      576
   Gln Ile Arg Leu Gln Glu Glu Gln Arg Ala Lys Glu Leu Tyr Trp Thr
               180                 185                 190
   ctg aag cag gcc cag ctg cac agc caa gcc agt gag aat gag gag cgt      624
   Leu Lys Gln Ala Gln Leu His Ser Gln Ala Ser Glu Asn Glu Glu Arg
           195                 200                 205
   gaa tgg gaa gaa cag ctg cgg aga tcc aag gct gct gat gaa gag agg      672
   Glu Trp Glu Glu Gln Leu Arg Arg Ser Lys Ala Ala Asp Glu Glu Arg
       210                 215                 220
   agc cgt cgt gct cag cga gcc cgg gat gag tac cgg cgc cat tca ctc      720
   Ser Arg Arg Ala Gln Arg Ala Arg Asp Glu Tyr Arg Arg His Ser Leu
   225                 230                 235                 240
   cga gcc atc cag aag ggc act gtt gct ggc cta agc acc atg ttc caa      768
   Arg Ala Ile Gln Lys Gly Thr Val Ala Gly Leu Ser Thr Met Phe Gln
                   245                 250                 255
   gag ctt ggc cag aac cac gag caa gag gca aga ctt tat cac caa ctt      816
   Glu Leu Gly Gln Asn His Glu Gln Glu Ala Arg Leu Tyr His Gln Leu
               260                 265                 270
   cct gac acc agt cca cca tca ccc ctc aca gga cct gac agg acc tgg      864
   Pro Asp Thr Ser Pro Pro Ser Pro Leu Thr Gly Pro Asp Arg Thr Trp
           275                 280                 285
   gag cga cct ctg cgt cca ctc tcc aga gaa gtc atc gtg cgc tgg ttc      912
   Glu Arg Pro Leu Arg Pro Leu Ser Arg Glu Val Ile Val Arg Trp Phe
       290                 295                 300
   aag gag gaa cag ctg cct cgc cga gca ggc ttt gag agg aac acc aag      960
   Lys Glu Glu Gln Leu Pro Arg Arg Ala Gly Phe Glu Arg Asn Thr Lys
   305                 310                 315                 320
   tcc atc gcc cct tgg ttt cat gga att att agc cga gag agt gca gaa     1008
   Ser Ile Ala Pro Trp Phe His Gly Ile Ile Ser Arg Glu Ser Ala Glu
                   325                 330                 335
   gac ctt ctg gag aat atg acc gag gga gca ttt ctg gtc cgg gtc agt     1056
   Asp Leu Leu Glu Asn Met Thr Glu Gly Ala Phe Leu Val Arg Val Ser
               340                 345                 350
   gag aag atc tgg ggt tat acc ctg tcc tac cgc ctg cag aga ggc ttc 1104
   Glu Lys Ile Trp Gly Tyr Thr Leu Ser Tyr Arg Leu Gln Arg Gly Phe
   355 360 365
   aaa cac ttc ctt gtg gat gct tct ggg gac ttc tac agc ttt ctg gga 1152
   Lys His Phe Leu Val Asp Ala Ser Gly Asp Phe Tyr Ser Phe Leu Gly
   370 375 380
   gtg gac cct aat cgc cat gcc acc cta aca gat ctc att gat ttc cac 1200
   Val Asp Pro Asn Arg His Ala Thr Leu Thr Asp Leu Ile Asp Phe His
   385 390 395 400
   aag gag gag atc atc act gtt tca ggg gga gag ttg cta cag gaa ccc 1248
   Lys Glu Glu Ile Ile Thr Val Ser Gly Gly Glu Leu Leu Gln Glu Pro
   405 410 415
   tgt gga cag agg gat agc cca cca gac tat cac ctg ttg ttt gaa tga 1296
   Cys Gly Gln Arg Asp Ser Pro Pro Asp Tyr His Leu Leu Phe Glu
   420 425 430
<210> 2
   <211> 431
   <212> PRT
   <213> Mus musculus
<400> 2
   Met Leu Gln Gln Ile Leu Gln Asp Met Tyr Ile Asp Pro Glu Leu Leu
   1 5 10 15
   Ala Glu Leu Ser Asp Val Gln Lys His Ile Leu Phe Tyr Lys Met Arg
   20 25 30
   Glu Glu Gln Leu Arg Arg Trp Arg Glu Arg Glu Ala Trp Asp Ala Leu
   35 40 45
   Ala Gln Ala Glu Gly Leu Arg Pro Ala Lys Val Lys Arg Ala Ser Asn
   50 55 60
   Lys His Leu Gln Trp Leu Leu Gly Ala Asp Gly Glu Val Trp Val Trp
   65 70 75 80
   Val Met Gly Glu Gly Pro Gly Asp Lys Pro Tyr Glu Glu Ile Ser Glu
   85 90 95
   Glu Leu Ile Ala Glu Arg Ala Arg Leu Gln Ala Gln Lys Glu Ala Glu
   100 105 110
   Glu Leu Trp Arg Gln Lys Glu Ala Glu Ile Thr Lys Lys Phe Arg Asp
   115 120 125
   Ala Leu Ala Asn Glu Lys Ala Arg Ile Leu Ala Glu Lys Trp Lys Val
   130 135 140
   Glu Met Glu Asp Arg Lys Ala Ala Lys Ile Leu Glu Glu Arg Ile His
   145 150 155 160
   Glu Glu Phe Lys Arg Lys Glu Glu Glu Glu Arg Arg Arg Gly Glu Glu
   165 170 175
   Gln Ile Arg Leu Gln Glu Glu Gln Arg Ala Lys Glu Leu Tyr Trp Thr
   180 185 190
   Leu Lys Gln Ala Gln Leu His Ser Gln Ala Ser Glu Asn Glu Glu Arg
   195 200 205
   Glu Trp Glu Glu Gln Leu Arg Arg Ser Lys Ala Ala Asp Glu Glu Arg
   210 215 220
   Ser Arg Arg Ala Gln Arg Ala Arg Asp Glu Tyr Arg Arg His Ser Leu
   225 230 235 240
   Arg Ala Ile Gln Lys Gly Thr Val Ala Gly Leu Ser Thr Met Phe Gln
   245 250 255
   Glu Leu Gly Gln Asn His Glu Gln Glu Ala Arg Leu Tyr His Gln Leu
   260 265 270
   Pro Asp Thr Ser Pro Pro Ser Pro Leu Thr Gly Pro Asp Arg Thr Trp
   275 280 285
   Glu Arg Pro Leu Arg Pro Leu Ser Arg Glu Val Ile Val Arg Trp Phe
   290 295 300
   Lys Glu Glu Gln Leu Pro Arg Arg Ala Gly Phe Glu Arg Asn Thr Lys
   305 310 315 320
   Ser Ile Ala Pro Trp Phe His Gly Ile Ile Ser Arg Glu Ser Ala Glu
   325 330 335
   Asp Leu Leu Glu Asn Met Thr Glu Gly Ala Phe Leu Val Arg Val Ser
   340 345 350
   Glu Lys Ile Trp Gly Tyr Thr Leu Ser Tyr Arg Leu Gln Arg Gly Phe
   355 360 365
   Lys His Phe Leu Val Asp Ala Ser Gly Asp Phe Tyr Ser Phe Leu Gly
   370 375 380
   Val Asp Pro Asn Arg His Ala Thr Leu Thr Asp Leu Ile Asp Phe His
   385 390 395 400
   Lys Glu Glu Ile Ile Thr Val Ser Gly Gly Glu Leu Leu Gln Glu Pro
   405 410 415
   Cys Gly Gln Arg Asp Ser Pro Pro Asp Tyr His Leu Leu Phe Glu
   420 425 430
<210> 3
   <211> 1302
   <212> DNA
   <213> Homo sapiens
<220>
   <221> CDS
   <222> (1) . . (302)
<400> 3
   atg ctg cag cag atc ctg cac gac atg tac atc gac ccc gag ctc ctt      48
   Met Leu Gln Gln Ile Leu His Asp Met Tyr Ile Asp Pro Glu Leu Leu
   1               5                   10                  15
   gcc gag ctc agc gat gtg cag aag cac atc ctc ttc tac aaa atg cgg      96
   Ala Glu Leu Ser Asp Val Gln Lys His Ile Leu Phe Tyr Lys Met Arg
               20                  25                  30
   gag gag cag ctg agg cgc tgg aag gag cgg gag act tgg gag gcc ctg      144
   Glu Glu Gln Leu Arg Arg Trp Lys Glu Arg Glu Thr Trp Glu Ala Leu
           35                  40                  45
   gcc cag gac gag ggt ctc agg cct cca aag acc aag cga gca gcg agt      192
   Ala Gln Asp Glu Gly Leu Arg Pro Pro Lys Thr Lys Arg Ala Ala Ser
       50                  55                  60
   gac aag cac atc caa tgg ctc cta ggg gca gat ggc gag gtc tgg gtc      240
   Asp Lys His Ile Gln Trp Leu Leu Gly Ala Asp Gly Glu Val Trp Val
   65                  70                  75                  80
   tgg atc atg gga gaa ggc cct ggt gac aag ccc tac gaa gag atc tct      288
   Trp Ile Met Gly Glu Gly Pro Gly Asp Lys Pro Tyr Glu Glu Ile Ser
                   85                  90                  95
   gag gag ctg att gca gag agg gcg cgg ctg cag gca cag agg gaa gct      336
   Glu Glu Leu Ile Ala Glu Arg Ala Arg Leu Gln Ala Gln Arg Glu Ala
               100                 105                 110
   gag gag ctc tgg aga cag aag gag gca gag atc acc aag aag ttc cgg      384
   Glu Glu Leu Trp Arg Gln Lys Glu Ala Glu Ile Thr Lys Lys Phe Arg
           115                 120                 125
   gat gct ctg gcc aat gag aaa gcc cgg atc ttg gcg gag aag tgg aaa      432
   Asp Ala Leu Ala Asn Glu Lys Ala Arg Ile Leu Ala Glu Lys Trp Lys
       130                 135                 140
   gtg gag atg gaa gac cgc aag gct gcc aaa gtc ctg gag gaa cgc atc      480
   Val Glu Met Glu Asp Arg Lys Ala Ala Lys Val Leu Glu Glu Arg Ile
   145                 150                 155                 160
   cac gag gaa ttc aag agg aaa gag gaa gag gag agg aag cga gga gaa      528
   His Glu Glu Phe Lys Arg Lys Glu Glu Glu Glu Arg Lys Arg Gly Glu
                   165                 170                 175
   gag cag att cgc ctc cag gaa gag cag agg gcg aag gag ctc tac tgg      576
   Glu Gln Ile Arg Leu Gln Glu Glu Gln Arg Ala Lys Glu Leu Tyr Trp
   180 185 190
   acc ctg aag cag gct cag ctg cat tgc caa gcc agt gag aaa gag gag 624
   Thr Leu Lys Gln Ala Gln Leu His Cys Gln Ala Ser Glu Lys Glu Glu
   195 200 205
   cga gag tgg gaa gaa cag ctg cgg aga tcc aag gcg gct gat gag gag 672
   Arg Glu Trp Glu Glu Gln Leu Arg Arg Ser Lys Ala Ala Asp Glu Glu
   210 215 220
   agg agc cgc cga gcc cag cgc gcc cgg gac gag tac cga cac cac tcg 720
   Arg Ser Arg Arg Ala Gln Arg Ala Arg Asp Glu Tyr Arg His His Ser
   225 230 235 240
   ctc cgt gct atc cag aag ggc acg gtc gct ggc ctc agc tcc atg ttc 768
   Leu Arg Ala Ile Gln Lys Gly Thr Val Ala Gly Leu Ser Ser Met Phe
   245 250 255
   cgg gag ctt ggc cag agc cat gag cag gag gca aga ctc tac cac cac 816
   Arg Glu Leu Gly Gln Ser His Glu Gln Glu Ala Arg Leu Tyr His His
   260 265 270
   ctc ccc gac ccg ggt ctg ccg cag ccc ctt gcc ctg ccg gtc agc agg 864
   Leu Pro Asp Pro Gly Leu Pro Gln Pro Leu Ala Leu Pro Val Ser Arg
   275 280 285
   acc tgg gag cgc ccg ctg cgc cca gtc tcc aga gat gtc atc gtc cgc 912
   Thr Trp Glu Arg Pro Leu Arg Pro Val Ser Arg Asp Val Ile Val Arg
   290 295 300
   tgg ttt aag gag gag cag ctg cct cgc cga gct ggc ttc gag agg aac 960
   Trp Phe Lys Glu Glu Gln Leu Pro Arg Arg Ala Gly Phe Glu Arg Asn
   305 310 315 320
   acc aag ttc atc gcc ccc tgg ttc cat gga att att agc cga gaa gat 1008
   Thr Lys Phe Ile Ala Pro Trp Phe His Gly Ile Ile Ser Arg Glu Asp
   325 330 335
   gca gaa gct ctc ctg gag aac atg act gag gga gca ttc ctg gtc cgg 1056
   Ala Glu Ala Leu Leu Glu Asn Met Thr Glu Gly Ala Phe Leu Val Arg
   340 345 350
   gtc agt gag aaa atc tgg ggt tac acc ctc tcc tac cgc ctg cag aaa 1104
   Val Ser Glu Lys Ile Trp Gly Tyr Thr Leu Ser Tyr Arg Leu Gln Lys
   355 360 365
   ggg ttc aaa cac ttt ctt gtg gat gct tct ggg gat ttt tac agc ttc 1152
   Gly Phe Lys His Phe Leu Val Asp Ala Ser Gly Asp Phe Tyr Ser Phe
   370 375 380
   ctg gga gtg gac ccc aat cgc cat gca acg ctc acg gat ctc gtt gat 1200
   Leu Gly Val Asp Pro Asn Arg His Ala Thr Leu Thr Asp Leu Val Asp
   385 390 395 400
   ttc cat aag gag gaa att atc act gtt tca gga gga gag tta ctt cag 1248
   Phe His Lys Glu Glu Ile Ile Thr Val Ser Gly Gly Glu Leu Leu Gln
                   405                 410                 415
   gaa ccc tgc gga cag agg gac agc cca cca gac tac cat ctg ttg ttt      1296
   Glu Pro Cys Gly Gln Arg Asp Ser Pro Pro Asp Tyr His Leu Leu Phe
               420                 425                 430
   gaa taa                                                              1302
   Glu
<210> 4
   <211> 433
   <212> PRT
   <213> Homo sapiens
<400> 4
   Met Leu Gln Gln Ile Leu His Asp Met Tyr Ile Asp Pro Glu Leu Leu
   1 5 10 15
   Ala Glu Leu Ser Asp Val Gln Lys His Ile Leu Phe Tyr Lys Met Arg
   20 25 30
   Glu Glu Gln Leu Arg Arg Trp Lys Glu Arg Glu Thr Trp Glu Ala Leu
   35 40 45
   Ala Gln Asp Glu Gly Leu Arg Pro Pro Lys Thr Lys Arg Ala Ala Ser
   50 55 60
   Asp Lys His Ile Gln Trp Leu Leu Gly Ala Asp Gly Glu Val Trp Val
   65 70 75 80
   Trp Ile Met Gly Glu Gly Pro Gly Asp Lys Pro Tyr Glu Glu Ile Ser
   85 90 95
   Glu Glu Leu Ile Ala Glu Arg Ala Arg Leu Gln Ala Gln Arg Glu Ala
   100 105 110
   Glu Glu Leu Trp Arg Gln Lys Glu Ala Glu Ile Thr Lys Lys Phe Arg
   115 120 125
   Asp Ala Leu Ala Asn Glu Lys Ala Arg Ile Leu Ala Glu Lys Trp Lys
   130 135 140
   Val Glu Met Glu Asp Arg Lys Ala Ala Lys Val Leu Glu Glu Arg Ile
   145 150 155 160
   His Glu Glu Phe Lys Arg Lys Glu Glu Glu Glu Arg Lys Arg Gly Glu
   165 170 175
   Glu Gln Ile Arg Leu Gln Glu Glu Gln Arg Ala Lys Glu Leu Tyr Trp
   180 185 190
   Thr Leu Lys Gln Ala Gln Leu His Cys Gln Ala Ser Glu Lys Glu Glu
   195 200 205
   Arg Glu Trp Glu Glu Gln Leu Arg Arg Ser Lys Ala Ala Asp Glu Glu
   210 215 220
   Arg Ser Arg Arg Ala Gln Arg Ala Arg Asp Glu Tyr Arg His His Ser
   225 230 235 240
   Leu Arg Ala Ile Gln Lys Gly Thr Val Ala Gly Leu Ser Ser Met Phe
   245 250 255
   Arg Glu Leu Gly Gln Ser His Glu Gln Glu Ala Arg Leu Tyr His His
   260 265 270
   Leu Pro Asp Pro Gly Leu Pro Gln Pro Leu Ala Leu Pro Val Ser Arg
   275 280 285
   Thr Trp Glu Arg Pro Leu Arg Pro Val Ser Arg Asp Val Ile Val Arg
   290 295 300
   Trp Phe Lys Glu Glu Gln Leu Pro Arg Arg Ala Gly Phe Glu Arg Asn
   305 310 315 320
   Thr Lys Phe Ile Ala Pro Trp Phe His Gly Ile Ile Ser Arg Glu Asp
   325 330 335
   Ala Glu Ala Leu Leu Glu Asn Met Thr Glu Gly Ala Phe Leu Val Arg
   340 345 350
   Val Ser Glu Lys Ile Trp Gly Tyr Thr Leu Ser Tyr Arg Leu Gln Lys
   355 360 365
   Gly Phe Lys His Phe Leu Val Asp Ala Ser Gly Asp Phe Tyr Ser Phe
   370 375 380
   Leu Gly Val Asp Pro Asn Arg His Ala Thr Leu Thr Asp Leu Val Asp
   385 390 395 400
   Phe His Lys Glu Glu Ile Ile Thr Val Ser Gly Gly Glu Leu Leu Gln
   405 410 415
   Glu Pro Cys Gly Gln Arg Asp Ser Pro Pro Asp Tyr His Leu Leu Phe
   420 425 430
   Glu
<210> 5
   <211> 1263
   <212> DNA
   <213> Mus musculus
<220>
   <221> CDS
   <222> (1)..(1263)
<400> 5
   atg ctg agg cag ata ctg tca gat atg ttc ata gac cct gac ctg ctg      48
   Met Leu Arg Gln Ile Leu Ser Asp Met Phe Ile Asp Pro Asp Leu Leu
   1               5                   10                  15
   gca gag ctc agc gaa gag cag aaa cag atc ttg ttc tac aag atg aga      96
   Ala Glu Leu Ser Glu Glu Gln Lys Gln Ile Leu Phe Tyr Lys Met Arg
               20                  25                  30
   gag gaa cag atc cga cga tgg aaa gaa aga gaa gcg gcc atg gaa aga      144
   Glu Glu Gln Ile Arg Arg Trp Lys Glu Arg Glu Ala Ala Met Glu Arg
           35                  40                  45
   aag gag tcc ttg cca gtg aaa tcc agg cca aaa aaa gag aat ggc aag      192
   Lys Glu Ser Leu Pro Val Lys Ser Arg Pro Lys Lys Glu Asn Gly Lys
       50                  55                  60
   tct gtc cat tgg aag ctg ggc gcc gat aag cag gtg tgg gtt tgg gta      240
   Ser Val His Trp Lys Leu Gly Ala Asp Lys Gln Val Trp Val Trp Val
   65                  70                  75                  80
   atg ggc gag cac cat ctg gac aaa ccc tat gat gtg ctg tgt gat gag      288
   Met Gly Glu His His Leu Asp Lys Pro Tyr Asp Val Leu Cys Asp Glu
                   85                  90                  95
   atc ctt gcg gag agg gag cat ctg aga gca gcg aag gat tca gag ctc      336
   Ile Leu Ala Glu Arg Glu His Leu Arg Ala Ala Lys Asp Ser Glu Leu
               100                 105                 110
   agg aaa act cag tct cta gaa ctc gcc aat agc tta aaa ata aag tca      384
   Arg Lys Thr Gln Ser Leu Glu Leu Ala Asn Ser Leu Lys Ile Lys Ser
           115                 120                 125
   cag aac tgt gat ctg caa gca atg aag aag aca gag cct cag aat gtc      432
   Gln Asn Cys Asp Leu Gln Ala Met Lys Lys Thr Glu Pro Gln Asn Val
       130                 135                 140
   acc agg aaa gca gct tca gaa gag gca tca ggt caa gga ccc aga gca      480
   Thr Arg Lys Ala Ala Ser Glu Glu Ala Ser Gly Gln Gly Pro Arg Ala
   145                 150                 155                 160
   ata cca acc agg aag gat gac aaa gcc caa act aaa ccc gtc aag gaa      528
   Ile Pro Thr Arg Lys Asp Asp Lys Ala Gln Thr Lys Pro Val Lys Glu
                   165                 170                 175
   aaa gac cac gag gaa atg aag cag aca gag gat gag aaa acc aag cag      576
   Lys Asp His Glu Glu Met Lys Gln Thr Glu Asp Glu Lys Thr Lys Gln
               180                 185                 190
   ata tac aag agc tgg aaa gaa gac tca gaa tgg caa gca tct ctg cga      624
   Ile Tyr Lys Ser Trp Lys Glu Asp Ser Glu Trp Gln Ala Ser Leu Arg
           195                 200                 205
   aaa tct aag gcg gct gat gag aag aga cgc tct tta gct aaa caa gca      672
   Lys Ser Lys Ala Ala Asp Glu Lys Arg Arg Ser Leu Ala Lys Gln Ala
                   210                 215                 220
   cgg gaa gac tac aag agg cta tcc caa agg ggg agg agt ggg gac gga      720
   Arg Glu Asp Tyr Lys Arg Leu Ser Gln Arg Gly Arg Ser Gly Asp Gly
   225                 230                 235                 240
   ctg cag aac cca ctg aca ggt cca cag aag ccc aga aga cct cct ctt      768
   Leu Gln Asn Pro Leu Thr Gly Pro Gln Lys Pro Arg Arg Pro Pro Leu
                   245                 250                 255
   cct ccg aag ccc cag ttc cta cag ccg ctg gga atc cct cca aag tct      816
   Pro Pro Lys Pro Gln Phe Leu Gln Pro Leu Gly Ile Pro Pro Lys Ser
               260                 265                 270
   tta ggg aat cag ggg gtg ata agg acc gag atc agc tcc gcc cag atg      864
   Leu Gly Asn Gln Gly Val Ile Arg Thr Glu Ile Ser Ser Ala Gln Met
           275                 280                 285
   gac acc att cga tgg ttc aaa gag gaa cag ttg cca ttc cgt gca ggt      912
   Asp Thr Ile Arg Trp Phe Lys Glu Glu Gln Leu Pro Phe Arg Ala Gly
       290                 295                 300
   tac cag aaa aac tca gac acc att gct cct tgg ttc cat ggg att ctc      960
   Tyr Gln Lys Asn Ser Asp Thr Ile Ala Pro Trp Phe His Gly Ile Leu
   305                 310                 315                 320
   aca ctg aag aaa gca aat gaa ctt ctg agc aca ggt gtg ccg gga agt     1008
   Thr Leu Lys Lys Ala Asn Glu Leu Leu Ser Thr Gly Val Pro Gly Ser
                   325                 330                 335
   ttt ttg att cga gtc agt gaa aag atc aag ggc tat gcc ctg tcc tac     1056
   Phe Leu Ile Arg Val Ser Glu Lys Ile Lys Gly Tyr Ala Leu Ser Tyr
               340                 345                 350
   ctg tct gag gaa ggc tgc aaa cat ttc ctt ata gat gca tct gcc aac     1104
   Leu Ser Glu Glu Gly Cys Lys His Phe Leu Ile Asp Ala Ser Ala Asn
           355                 360                 365
   tct tac agc ttc ctg ggt gtg gac cag ctg cag cat gct aca ctg gca     1152
   Ser Tyr Ser Phe Leu Gly Val Asp Gln Leu Gln His Ala Thr Leu Ala
       370                 375                 380
   gat ttg gtg gaa tat cac aag gag gag ccc ata acc tct ctg ggg aag     1200
   Asp Leu Val Glu Tyr His Lys Glu Glu Pro Ile Thr Ser Leu Gly Lys
   385                 390                 395                 400
   gaa ctc ctt ctg tac ccc tgt ggt caa caa gac aag ctg ccc gac tac     1248
   Glu Leu Leu Leu Tyr Pro Cys Gly Gln Gln Asp Lys Leu Pro Asp Tyr
                   405                 410                 415
   ctg gag ctc ttc cag                                                 1263
   Leu Glu Leu Phe Gln
               420
<210> 6
   <211> 421
   <212> PRT
   <213> Mus musculus
<400> 6
   Met Leu Arg Gln Ile Leu Ser Asp Met Phe Ile Asp Pro Asp Leu Leu
   1               5                   10                  15
   Ala Glu Leu Ser Glu Glu Gln Lys Gln Ile Leu Phe Tyr Lys Met Arg
               20                  25                  30
   Glu Glu Gln Ile Arg Arg Trp Lys Glu Arg Glu Ala Ala Met Glu Arg
           35                  40                  45
   Lys Glu Ser Leu Pro Val Lys Ser Arg Pro Lys Lys Glu Asn Gly Lys
       50                  55                  60
   Ser Val His Trp Lys Leu Gly Ala Asp Lys Gln Val Trp Val Trp Val
   65                  70                  75                  80
   Met Gly Glu His His Leu Asp Lys Pro Tyr Asp Val Leu Cys Asp Glu
                   85                  90                  95
   Ile Leu Ala Glu Arg Glu His Leu Arg Ala Ala Lys Asp Ser Glu Leu
               100                 105                 110
   Arg Lys Thr Gln Ser Leu Glu Leu Ala Asn Ser Leu Lys Ile Lys Ser
           115                 120                 125
   Gln Asn Cys Asp Leu Gln Ala Met Lys Lys Thr Glu Pro Gln Asn Val
       130                 135                 140
   Thr Arg Lys Ala Ala Ser Glu Glu Ala Ser Gly Gln Gly Pro Arg Ala
   145                 150                 155                 160
   Ile Pro Thr Arg Lys Asp Asp Lys Ala Gln Thr Lys Pro Val Lys Glu
                   165                 170                 175
   Lys Asp His Glu Glu Met Lys Gln Thr Glu Asp Glu Lys Thr Lys Gln
               180                 185                 190
   Ile Tyr Lys Ser Trp Lys Glu Asp Ser Glu Trp Gln Ala Ser Leu Arg
           195                 200                 205
   Lys Ser Lys Ala Ala Asp Glu Lys Arg Arg Ser Leu Ala Lys Gln Ala
       210                 215                 220
   Arg Glu Asp Tyr Lys Arg Leu Ser Gln Arg Gly Arg Ser Gly Asp Gly
   225                 230                 235                 240
   Leu Gln Asn Pro Leu Thr Gly Pro Gln Lys Pro Arg Arg Pro Pro Leu
                   245                 250                 255
   Pro Pro Lys Pro Gln Phe Leu Gln Pro Leu Gly Ile Pro Pro Lys Ser
   260 265 270
   Leu Gly Asn Gln Gly Val Ile Arg Thr Glu Ile Ser Ser Ala Gln Met
   275 280 285
   Asp Thr Ile Arg Trp Phe Lys Glu Glu Gln Leu Pro Phe Arg Ala Gly
   290 295 300
   Tyr Gln Lys Asn Ser Asp Thr Ile Ala Pro Trp Phe His Gly Ile Leu
   305 310 315 320
   Thr Leu Lys Lys Ala Asn Glu Leu Leu Ser Thr Gly Val Pro Gly Ser
   325 330 335
   Phe Leu Ile Arg Val Ser Glu Lys Ile Lys Gly Tyr Ala Leu Ser Tyr
   340 345 350
   Leu Ser Glu Glu Gly Cys Lys His Phe Leu Ile Asp Ala Ser Ala Asn
   355 360 365
   Ser Tyr Ser Phe Leu Gly Val Asp Gln Leu Gln His Ala Thr Leu Ala
   370 375 380
   Asp Leu Val Glu Tyr His Lys Glu Glu Pro Ile Thr Ser Leu Gly Lys
   385 390 395 400
   Glu Leu Leu Leu Tyr Pro Cys Gly Gln Gln Asp Lys Leu Pro Asp Tyr
   405 410 415
   Leu Glu Leu Phe Gln
   420
<210> 7
   <211> 1365
   <212> DNA
   <213> Homo sapiens
<220>
   <221> CDS
   <222> (1)..(1365)
<400> 7
   atg ctg aaa cag ata ctg tcg gag atg tac ata gat cct gat cta ctg 48
   Met Leu Lys Gln Ile Leu Ser Glu Met Tyr Ile Asp Pro Asp Leu Leu
   1 5 10 15
   gca gag ctc agc gaa gaa cag aaa cag atc ctg ttc ttc aag atg aga 96
   Ala Glu Leu Ser Glu Glu Gln Lys Gln Ile Leu Phe Phe Lys Met Arg
   20 25 30
   gag gaa cag atc cga cga tgg aaa gaa aga gaa gca gct atg gaa aga 144
   Glu Glu Gln Ile Arg Arg Trp Lys Glu Arg Glu Ala Ala Met Glu Arg
   35 40 45
   aag gag tcc ctg cca gtg aaa ccc aga cca aag aaa gag aat ggc aaa      192
   Lys Glu Ser Leu Pro Val Lys Pro Arg Pro Lys Lys Glu Asn Gly Lys
       50                  55                  60
   tcg gtt cat tgg aaa ctt gga gct gat aag gaa gtc tgg gta tgg gtg      240
   Ser Val His Trp Lys Leu Gly Ala Asp Lys Glu Val Trp Val Trp Val
   65                  70                  75                  80
   atg ggc gaa cac cat cta gat aaa ccc tat gat gtg ctc tgt aat gaa      288
   Met Gly Glu His His Leu Asp Lys Pro Tyr Asp Val Leu Cys Asn Glu
                   85                  90                  95
   att att gct gag agg gcc cgg ctg aaa gca gaa cag gag gca gaa gag      336
   Ile Ile Ala Glu Arg Ala Arg Leu Lys Ala Glu Gln Glu Ala Glu Glu
               100                 105                 110
   ccc aga aaa act cac tct gaa gaa ttc acc aat agc ttg aaa aca aaa      384
   Pro Arg Lys Thr His Ser Glu Glu Phe Thr Asn Ser Leu Lys Thr Lys
           115                 120                 125
   tca cag tac cat gat ctg cag gct ccg gat aac cag cag act aaa gac      432
   Ser Gln Tyr His Asp Leu Gln Ala Pro Asp Asn Gln Gln Thr Lys Asp
       130                 135                 140
   atc tgg aag aaa gtg gca gaa aag gag gaa ctg gag caa gga tct agg      480
   Ile Trp Lys Lys Val Ala Glu Lys Glu Glu Leu Glu Gln Gly Ser Arg
   145                 150                 155                 160
   cca gca cca acc ctg gaa gaa gag aaa atc cga tca ctc tcc agt tct      528
   Pro Ala Pro Thr Leu Glu Glu Glu Lys Ile Arg Ser Leu Ser Ser Ser
                   165                 170                 175
   tca aga aat att caa caa atg ttg gca gat tca atc aat cgt atg aag      576
   Ser Arg Asn Ile Gln Gln Met Leu Ala Asp Ser Ile Asn Arg Met Lys
               180                 185                 190
   gca tat gca ttt cac cag aag aaa gaa tct atg aag aaa aaa caa gat      624
   Ala Tyr Ala Phe His Gln Lys Lys Glu Ser Met Lys Lys Lys Gln Asp
           195                 200                 205
   gaa gaa ata aat caa ata gaa gaa gag aga acg aag cag att tgt aag      672
   Glu Glu Ile Asn Gln Ile Glu Glu Glu Arg Thr Lys Gln Ile Cys Lys
       210                 215                 220
   agc tgg aaa gaa gac tcg gaa tgg cag gca tct ctg cga aaa tcc aaa      720
   Ser Trp Lys Glu Asp Ser Glu Trp Gln Ala Ser Leu Arg Lys Ser Lys
   225                 230                 235                 240
   gca gct gat gag aag aga cgc tcc ttg gct aaa caa gca cga gaa gac      768
   Ala Ala Asp Glu Lys Arg Arg Ser Leu Ala Lys Gln Ala Arg Glu Asp
                   245                 250                 255
   tac aag agg ttg tcc ctc ggg gcc cag aaa gga aga ggc ggt gag agg      816
   Tyr Lys Arg Leu Ser Leu Gly Ala Gln Lys Gly Arg Gly Gly Glu Arg
               260                 265                 270
   ctg caa agc ccc ttg cgt gtt ccg cag aaa cca gaa aga cct ccc ctt      864
   Leu Gln Ser Pro Leu Arg Val Pro Gln Lys Pro Glu Arg Pro Pro Leu
           275                 280                 285
   cca ccc aag cct cag ttc cta aac tca ggg gca tat cct caa aaa cct      912
   Pro Pro Lys Pro Gln Phe Leu Asn Ser Gly Ala Tyr Pro Gln Lys Pro
       290                 295                 300
   ctt aga aat cag gga gtg gtg agg aca ctg tcc agc tct gcc caa gag      960
   Leu Arg Asn Gln Gly Val Val Arg Thr Leu Ser Ser Ser Ala Gln Glu
   305                 310                 315                 320
   gac atc atc cgg tgg ttt aaa gag gag cag cta cca ctt cga gcg ggc     1008
   Asp Ile Ile Arg Trp Phe Lys Glu Glu Gln Leu Pro Leu Arg Ala Gly
                   325                 330                 335
   tac cag aaa acc tca gac acc ata gcc ccc tgg ttc cat gga att ctc     1056
   Tyr Gln Lys Thr Ser Asp Thr Ile Ala Pro Trp Phe His Gly Ile Leu
               340                 345                 350
   aca ctc aag aaa gca aat gaa ctt ctt ctg agc aca ggc atg ccc ggc     1104
   Thr Leu Lys Lys Ala Asn Glu Leu Leu Leu Ser Thr Gly Met Pro Gly
           355                 360                 365
   agt ttt ctc atc cga gtc agt gaa agg atc aaa ggc tat gcc ctg tcc     1152
   Ser Phe Leu Ile Arg Val Ser Glu Arg Ile Lys Gly Tyr Ala Leu Ser
       370                 375                 380
   tat ctg tcg gag gac ggc tgt aaa cat ttc ctc atc gat gcc tct gca     1200
   Tyr Leu Ser Glu Asp Gly Cys Lys His Phe Leu Ile Asp Ala Ser Ala
   385                 390                 395                 400
   gac gcc tac agc ttc ctg ggc gtg gac cag cta cag cat gcc acc ttg     1248
   Asp Ala Tyr Ser Phe Leu Gly Val Asp Gln Leu Gln His Ala Thr Leu
                   405                 410                 415
   gcg gat ttg gtg gaa tat cac aag gag gaa ccc atc act tcc ctg ggg     1296
   Ala Asp Leu Val Glu Tyr His Lys Glu Glu Pro Ile Thr Ser Leu Gly
               420                 425                 430
   aag gag ctc ctt ctc tat ccc tgt ggt cag cag gac cag ctg cct gac     1344
   Lys Glu Leu Leu Leu Tyr Pro Cys Gly Gln Gln Asp Gln Leu Pro Asp
           435                 440                 445
   tac ctg gag ctg ttt gag tga                                         1365
   Tyr Leu Glu Leu Phe Glu
       450
<210> 8
   <211> 454
   <212> PRT
   <213> Homo sapiens
<400> 8
   Met Leu Lys Gln Ile Leu Ser Glu Met Tyr Ile Asp Pro Asp Leu Leu
   1               5                   10                  15
   Ala Glu Leu Ser Glu Glu Gln Lys Gln Ile Leu Phe Phe Lys Met Arg
               20                  25                  30
   Glu Glu Gln Ile Arg Arg Trp Lys Glu Arg Glu Ala Ala Met Glu Arg
           35                  40                  45
   Lys Glu Ser Leu Pro Val Lys Pro Arg Pro Lys Lys Glu Asn Gly Lys
       50                  55                  60
   Ser Val His Trp Lys Leu Gly Ala Asp Lys Glu Val Trp Val Trp Val
   65                  70                  75                  80
   Met Gly Glu His His Leu Asp Lys Pro Tyr Asp Val Leu Cys Asn Glu
                   85                  90                  95
   Ile Ile Ala Glu Arg Ala Arg Leu Lys Ala Glu Gln Glu Ala Glu Glu
               100                 105                 110
   Pro Arg Lys Thr His Ser Glu Glu Phe Thr Asn Ser Leu Lys Thr Lys
           115                 120                 125
   Ser Gln Tyr His Asp Leu Gln Ala Pro Asp Asn Gln Gln Thr Lys Asp
       130                 135                 140
   Ile Trp Lys Lys Val Ala Glu Lys Glu Glu Leu Glu Gln Gly Ser Arg
   145                 150                 155                 160
   Pro Ala Pro Thr Leu Glu Glu Glu Lys Ile Arg Ser Leu Ser Ser Ser
                   165                 170                 175
   Ser Arg Asn Ile Gln Gln Met Leu Ala Asp Ser Ile Asn Arg Met Lys
               180                 185                 190
   Ala Tyr Ala Phe His Gln Lys Lys Glu Ser Met Lys Lys Lys Gln Asp
           195                 200                 205
   Glu Glu Ile Asn Gln Ile Glu Glu Glu Arg Thr Lys Gln Ile Cys Lys
       210                 215                 220
   Ser Trp Lys Glu Asp Ser Glu Trp Gln Ala Ser Leu Arg Lys Ser Lys
   225                 230                 235                 240
   Ala Ala Asp Glu Lys Arg Arg Ser Leu Ala Lys Gln Ala Arg Glu Asp
                   245                 250                 255
   Tyr Lys Arg Leu Ser Leu Gly Ala Gln Lys Gly Arg Gly Gly Glu Arg
               260                 265                 270
   Leu Gln Ser Pro Leu Arg Val Pro Gln Lys Pro Glu Arg Pro Pro Leu
           275                 280                 285
   Pro Pro Lys Pro Gln Phe Leu Asn Ser Gly Ala Tyr Pro Gln Lys Pro
   290 295 300
   Leu Arg Asn Gln Gly Val Val Arg Thr Leu Ser Ser Ser Ala Gln Glu
   305 310 315 320
   Asp Ile Ile Arg Trp Phe Lys Glu Glu Gln Leu Pro Leu Arg Ala Gly
   325 330 335
   Tyr Gln Lys Thr Ser Asp Thr Ile Ala Pro Trp Phe His Gly Ile Leu
   340 345 350
   Thr Leu Lys Lys Ala Asn Glu Leu Leu Leu Ser Thr Gly Met Pro Gly
   355 360 365
   Ser Phe Leu Ile Arg Val Ser Glu Arg Ile Lys Gly Tyr Ala Leu Ser
   370 375 380
   Tyr Leu Ser Glu Asp Gly Cys Lys His Phe Leu Ile Asp Ala Ser Ala
   385 390 395 400
   Asp Ala Tyr Ser Phe Leu Gly Val Asp Gln Leu Gln His Ala Thr Leu
   405 410 415
   Ala Asp Leu Val Glu Tyr His Lys Glu Glu Pro Ile Thr Ser Leu Gly
   420 425 430
   Lys Glu Leu Leu Leu Tyr Pro Cys Gly Gln Gln Asp Gln Leu Pro Asp
   435 440 445
   Tyr Leu Glu Leu Phe Glu
   450
<210> 9
   <211> 180
   <212> PRT
   <213> Homo sapiens
<400> 9
   Met Leu Gln Gln Ile Leu His Asp Met Tyr Ile Asp Pro Glu Leu Leu
   1 5 10 15
   Ala Glu Leu Ser Asp Val Gln Lys His Ile Leu Phe Tyr Lys Met Arg
   20 25 30
   Glu Glu Gln Leu Arg Arg Trp Lys Glu Arg Glu Thr Trp Glu Ala Leu
   35 40 45
   Ala Gln Asp Glu Gly Leu Arg Pro Pro Lys Thr Lys Arg Ala Ala Ser
   50 55 60
   Asp Lys His Ile Gln Trp Leu Leu Gly Ala Asp Gly Glu Val Trp Val
   65 70 75 80
   Trp Ile Met Gly Glu Gly Pro Gly Asp Lys Pro Tyr Glu Glu Ile Ser
   85 90 95
   Glu Glu Leu Ile Ala Glu Arg Ala Arg Leu Gln Ala Gln Arg Glu Ala
   100 105 110
   Glu Glu Leu Trp Arg Gln Lys Glu Ala Glu Ile Thr Lys Lys Phe Arg
   115 120 125
   Asp Ala Leu Ala Asn Glu Lys Ala Arg Ile Leu Ala Glu Lys Trp Lys
   130 135 140
   Val Glu Met Glu Asp Arg Lys Ala Ala Lys Val Leu Glu Glu Arg Ile
   145 150 155 160
   His Glu Glu Phe Lys Arg Lys Glu Glu Glu Glu Arg Lys Arg Gly Glu
   165 170 175
   Glu Gln Ile Arg
   180
<210> 10
   <211> 179
   <212> PRT
   <213> Mus musculus
<400> 10
   Met Leu Gln Gln Ile Leu Gln Asp Met Tyr Ile Asp Pro Glu Leu Leu
   1               5                   10                  15
   Ala Glu Leu Ser Asp Val Gln Lys His Ile Leu Phe Tyr Lys Met Arg
               20                  25                  30
   Glu Glu Gln Leu Arg Arg Trp Arg Glu Arg Glu Ala Trp Asp Ala Leu
           35                  40                  45
   Ala Gln Ala Glu Gly Leu Arg Pro Ala Lys Val Lys Arg Ala Ser Asn
       50                  55                  60
   Lys His Leu Gln Trp Leu Leu Gly Ala Asp Gly Glu Val Trp Val Trp
   65                  70                  75                  80
   Val Met Gly Glu Gly Pro Gly Asp Lys Pro Tyr Glu Glu Ile Ser Glu
                   85                  90                  95
   Glu Leu Ile Ala Glu Arg Ala Arg Leu Gln Ala Gln Lys Glu Ala Glu
               100                 105                 110
   Glu Leu Trp Arg Gln Lys Glu Ala Glu Ile Thr Lys Lys Phe Arg Asp
           115                 120                 125
   Ala Leu Ala Asn Glu Lys Ala Arg Ile Leu Ala Glu Lys Trp Lys Val
       130                 135                 140
   Glu Met Glu Asp Arg Lys Ala Ala Lys Ile Leu Glu Glu Arg Ile His
   145                 150                 155                 160
   Glu Glu Phe Lys Arg Lys Glu Glu Glu Glu Arg Arg Arg Gly Glu Glu
                   165                 170                 175
   Gln Ile Arg
<210> 11
   <211> 178
   <212> PRT
   <213> Homo sapiens
<400> 11
   Met Leu Lys Gln Ile Leu Ser Glu Met Tyr Ile Asp Pro Asp Leu Leu
   1               5                   10                  15
   Ala Glu Leu Ser Glu Glu Gln Lys Gln Ile Leu Phe Phe Lys Met Arg
               20                  25                  30
   Glu Glu Gln Ile Arg Arg Trp Lys Glu Arg Glu Ala Ala Met Glu Arg
           35                  40                  45
   Lys Glu Ser Leu Pro Val Lys Pro Arg Pro Lys Lys Glu Asn Gly Lys
       50                  55                  60
   Ser Val His Trp Lys Leu Gly Ala Asp Lys Glu Val Trp Val Trp Val
   65                  70                  75                  80
   Met Gly Glu His His Leu Asp Lys Pro Tyr Asp Val Leu Cys Asn Glu
                   85                  90                  95
   Ile Ile Ala Glu Arg Ala Arg Leu Lys Ala Glu Gln Glu Ala Glu Glu
               100                 105                 110
   Pro Arg Lys Thr His Ser Glu Glu Phe Thr Asn Ser Leu Lys Thr Lys
           115                 120                 125
   Ser Gln Tyr His Asp Leu Gln Ala Pro Asp Asn Gln Gln Thr Lys Asp
       130                 135                 140
   Ile Trp Lys Lys Val Ala Glu Lys Glu Glu Leu Glu Gln Gly Ser Arg
   145                 150                 155                 160
   Pro Ala Pro Thr Leu Glu Glu Glu Lys Ile Arg Ser Leu Ser Ser Ser
                   165                 170                 175
   Ser Arg
<210> 12
   <211> 173
   <212> PRT
   <213> Mus musculus
<400> 12
   Met Leu Arg Gln Ile Leu Ser Asp Met Phe Ile Asp Pro Asp Leu Leu
   1 5 10 15
   Ala Glu Leu Ser Glu Glu Gln Lys Gln Ile Leu Phe Tyr Lys Met Arg
   20 25 30
   Glu Glu Gln Ile Arg Arg Trp Lys Glu Arg Glu Ala Ala Met Glu Arg
   35 40 45
   Lys Glu Ser Leu Pro Val Lys Ser Arg Pro Lys Lys Glu Asn Gly Lys
   50 55 60
   Ser Val His Trp Lys Leu Gly Ala Asp Lys Gln Val Trp Val Trp Val
   65 70 75 80
   Met Gly Glu His His Leu Asp Lys Pro Tyr Asp Val Leu Cys Asp Glu
   85 90 95
   Ile Leu Ala Glu Arg Glu His Leu Arg Ala Ala Lys Asp Ser Glu Leu
   100 105 110
   Arg Lys Thr Gln Ser Leu Glu Leu Ala Asn Ser Leu Lys Ile Lys Ser
   115 120 125
   Gln Asn Cys Asp Leu Gln Ala Met Lys Lys Thr Glu Pro Gln Asn Val
   130 135 140
   Thr Arg Lys Ala Ala Ser Glu Glu Ala Ser Gly Gln Gly Pro Arg Ala
   145 150 155 160
   Ile Pro Thr Arg Lys Asp Asp Lys Ala Gln Thr Lys Pro
   165 170
<210> 13
   <211> 93
   <212> PRT
   <213> Danio rerio
<400> 13
   Met Leu Gln Gln Ile Leu Lys Asp Met Tyr Ile Asp Pro Asp Val Leu
   1 5 10 15
   Glu Ala Leu Asn Asp Glu Gln Lys Lys Met Leu Phe Leu Lys Met Arg
   20 25 30
   Glu Glu His Val Arg Arg Trp Lys Glu Arg Glu Glu Lys Leu Glu Arg
   35 40 45
   Glu Pro Leu Lys Pro Lys Ala Lys Thr Ala His Ser Lys Ser Val Ser
   50 55 60
   Trp Leu Leu Gly Arg Asp Gly Asp Val Gln Val Ile Val Ile Gly Glu
   65 70 75 80
   Met Asp Glu Phe Lys Ser Ser Lys Ile Ile Tyr Ser Gly
   85 90
<210> 14
   <211> 139
   <212> PRT
   <213> Danio rerio
<220>
   <221> misc_feature
   <222> (128)..()
   <223> X is unknown
<400> 14
   Met Leu Gln Gln Ile Leu Ala Asp Met Tyr Ile Asp Pro Asp Val Leu
   1 5 10 15
   Glu Ala Leu Asn Glu Glu Gln Lys Lys Ile Leu Phe Phe Lys Met Arg
   20 25 30
   Glu Glu Gln Val Arg Arg Trp Lys Glu Arg Glu Glu Gln Glu Ser Lys
   35 40 45
   Gly Glu Ile Lys Lys Glu Lys Leu Arg Lys Lys Lys Gly Pro Cys Lys
   50 55 60
   Asn Val Ser Trp Leu Leu Gly Arg Asp Gly Asp Val His Val Cys Ile
   65 70 75 80
   Ile Gly Glu Ser Asp Val Leu Glu Ser Pro Lys Leu Ile Leu Ser Glu
   85 90 95
   Leu Arg Asn Asn Thr Thr Ala Asn Gly Asn Asn Ile Asn Arg Ala Asn
   100 105 110
   Ala Glu Ser Ile Lys Ser Ser Ser Ile Lys Leu Asn Arg Val Gln Xaa
   115 120 125
   Thr Ser Thr Glu Pro Gly Ile Gln Leu Leu Leu
   130 135
<210> 15
   <211> 107
   <212> PRT
   <213> Homo sapiens
<400> 15
   Trp Phe His Gly Ile Ile Ser Arg Glu Asp Ala Glu Ala Leu Leu Glu
   1 5 10 15
   Asn Met Thr Glu Gly Ala Phe Leu Val Arg Val Ser Glu Lys Ile Trp
   20 25 30
   Gly Tyr Thr Leu Ser Tyr Arg Leu Gln Lys Gly Phe Lys His Phe Leu
   35 40 45
   Val Asp Ala Ser Gly Asp Phe Tyr Ser Phe Leu Gly Val Asp Pro Asn
   50 55 60
   Arg His Ala Thr Leu Thr Asp Leu Val Asp Phe His Lys Glu Glu Ile
   65 70 75 80
   Ile Thr Val Ser Gly Gly Glu Leu Leu Gln Glu Pro Cys Gly Gln Arg
   85 90 95
   Asp Ser Pro Pro Asp Tyr His Leu Leu Phe Glu
   100 105
<210> 16
   <211> 107
   <212> PRT
   <213> Mus musculus
<400> 16
   Trp Phe His Gly Ile Ile Ser Arg Glu Ser Ala Glu Asp Leu Leu Glu
   1 5 10 15
   Asn Met Thr Glu Gly Ala Phe Leu Val Arg Val Ser Glu Lys Ile Trp
   20 25 30
   Gly Tyr Thr Leu Ser Tyr Arg Leu Gln Arg Gly Phe Lys His Phe Leu
   35 40 45
   Val Asp Ala Ser Gly Asp Phe Tyr Ser Phe Leu Gly Val Asp Pro Asn
   50 55 60
   Arg His Ala Thr Leu Thr Asp Leu Ile Asp Phe His Lys Glu Glu Ile
   65 70 75 80
   Ile Thr Val Ser Gly Gly Glu Leu Leu Gln Glu Pro Cys Gly Gln Arg
   85 90 95
   Asp Ser Pro Pro Asp Tyr His Leu Leu Phe Glu
   100 105
<210> 17
   <211> 108
   <212> PRT
   <213> Homo sapiens
<400> 17
   Trp Phe His Gly Ile Leu Thr Leu Lys Lys Ala Asn Glu Leu Leu Leu
   1 5 10 15
   Ser Thr Gly Met Pro Gly Ser Phe Leu Ile Arg Val Ser Glu Arg Ile
   20 25 30
   Lys Gly Tyr Ala Leu Ser Tyr Leu Ser Glu Asp Gly Cys Lys His Phe
   35 40 45
   Leu Ile Asp Ala Ser Ala Asp Ala Tyr Ser Phe Leu Gly Val Asp Gln
   50 55 60
   Leu Gln His Ala Thr Leu Ala Asp Leu Val Glu Tyr His Lys Glu Glu
   65 70 75 80
   Pro Ile Thr Ser Leu Gly Lys Glu Leu Leu Leu Tyr Pro Cys Gly Gln
   85 90 95
   Gln Asp Gln Leu Pro Asp Tyr Leu Glu Leu Phe Glu
   100 105
<210> 18
   <211> 107
   <212> PRT
   <213> Mus musculus
<400> 18
   Trp Phe His Gly Ile Leu Thr Leu Lys Lys Ala Asn Glu Leu Leu Ser
   1 5 10 15
   Thr Gly Val Pro Gly Ser Phe Leu Ile Arg Val Ser Glu Lys Ile Lys
   20 25 30
   Gly Tyr Ala Leu Ser Tyr Leu Ser Glu Glu Gly Cys Lys His Phe Leu
   35 40 45
   Ile Asp Ala Ser Ala Asn Ser Tyr Ser Phe Leu Gly Val Asp Gln Leu
   50 55 60
   Gln His Ala Thr Leu Ala Asp Leu Val Glu Tyr His Lys Glu Glu Pro
   65 70 75 80
   Ile Thr Ser Leu Gly Lys Glu Leu Leu Leu Tyr Pro Cys Gly Gln Gln
   85 90 95
   Asp Lys Leu Pro Asp Tyr Leu Glu Leu Phe Gln
   100 105
<210> 19
   <211> 24
   <212> PRT
   <213> Mus musculus
<400> 19
   Cys Leu Pro Asp Thr Ser Pro Pro Ser Pro Leu Thr Gly Pro Asp Arg
   1 5 10 15
   Thr Trp Glu Arg Pro Leu Arg Cys
   20
<210> 20
   <211> 17
   <212> PRT
   <213> Mus musculus
<400> 20
   Cys Gly Glu Gly Pro Gly Asp Lys Pro Tyr Glu Glu Ile Ser Glu Glu
   1 5 10 15
   Cys
<210> 21
   <211> 19
   <212> PRT
   <213> Mus musculus
<400> 21
   Ala Asp Glu Glu Arg Ser Arg Arg Ala Gln Arg Ala Arg Asp Glu Tyr
   1 5 10 15
   Arg Arg Cys
<210> 22
<211> 19
<212> PRT
<213> Mus musculus
<400> 22
   Cys Gly Leu Arg Pro Pro Lys Thr Lys Arg Ala Ala Ser Asp Lys His
   1 5 10 15
   Ile Gln Cys
<210> 2 3
<211> 18
<212> PRT
<213> Mus musculus
<400> 23
<210> 24
<211> 13
<212> PRT
<213> Mus musculus
<400> 24
   Gln Gln Met Leu Ala Asp Ser Ile Asn Arg Met Lys Cys
   1 5 10
<210> 25
<211> 20
<212> DNA
<213> Artificial sequence
<220>
<223> Primer
<400> 25
   tgctgcagca gatcctgcac 20
<210> 26
   <211> 19
<212> DNA
<213> Artificial sequence
<220>
<223> Primer
<400> 26
   cctgaagtaa ctctcctcc 19
<210> 27
<211> 21
<212> DNA
<213> Artificial sequence
<220>
<223> Primer
<400> 27
   ccctggtgac aagccctacg a 21
<210> 28
   <211> 22
<212> DNA
<213> Artificial sequence
<220>
<223> Primer
<400> 28
   atagcacgga gcgagtggtg tc 22
<210> 29
<211> 18
<212> DNA
<213> Artificial sequence
<220>
<223> Primer
<400> 29
   tcactctgaa gaattcac 18
<210> 30
   <211> 18
<212> DNA
<213> Artificial sequence
<220>
<223> Primer
<400> 30
   tgagtgtgag aattccat 18
<210> 31
<211> 18
<212> DNA
<213> Artificial sequence
<220>
<223> Primer
<400> 31
   gatcactctc cagttctt 18
<210> 32
<211> 21
<212> DNA
<213> Artificial sequence
<220>
<223> Primer
<400> 32
   ggattttcgc agagatgcct g 21

## Description

### Field of the Invention

The present invention relates to a novel signalling protein associated with healthy, neoplastic and oncogenic tissues. The invention also relates to screening methods for identifying modulators of activity and/or expression of the signalling protein and to associated methods of diagnosis, prognosis and therapy of cancer.

### Background to the Invention

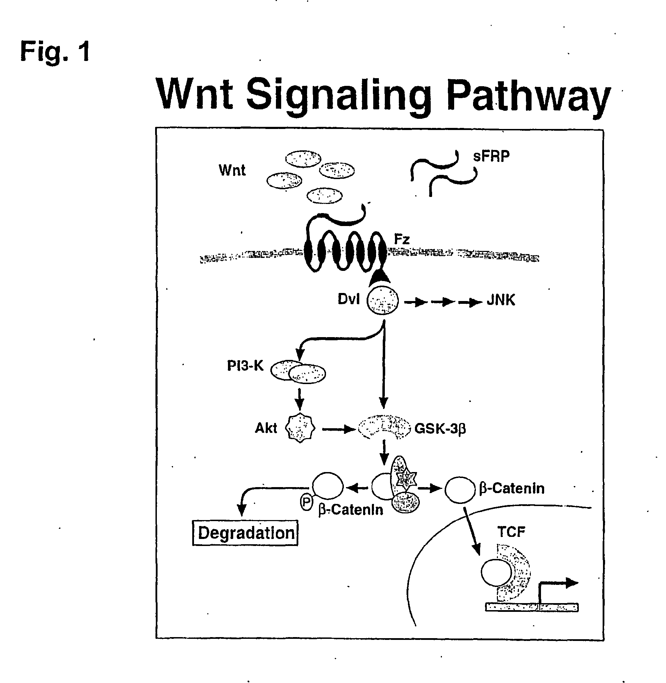

Human cancer results from the accumulation of independent genetic alterations which effect the transcriptional programs normally responsible for controlling cell growth and survival. Cancers within a single clinical category may exhibit seemingly disparate genetic defects that are, however, part of a common signal transduction pathway. The discovery of the wnt signalling pathway (summarised in Figure 1) and the structure/function analysis of its distinct molecular components has provided an outstanding example for the identification of common denominators in oncogenesis (Polakis (2000) Genes & Development 14:1837-1851; Peifer and Polakis (2000) Science 287: 1606-1609).

Wnt signalling is initiated by members of the family of secreted wnt glycoproteins which bind to one or several of their specific cell surface receptors, designated frizzled. This family of seven-path-transmembrane receptors activate the dishevelled protein upon binding of their respective ligand. Associated with axin, dishevelled prevents glycogen synthase kinase-3β from phosphorylating critical substrates such as β-catenin. Other substrates include the negative regulators axin itself and APC. Unphosphorylated β-catenin escapes degradation via the ubiquitin pathway and translocates to the nucleus where it associates with transcription factors such as T-cell factor (TCF) and leucocyte enhancer factor (LEF). In mammals, the number of identified target genes transcriptionally regulated by way of wnt signalling is still limited but includes c-myc, cyclin D1, c-jun, matrix metalloproteinases and CD44.

There have been numerous reports on transcipional overexpression, and sometimes underexpression, of wnt genes in human cancers but mRNA expression levels are merely correlative. More compelling evidence for the involvement of wnt-mediated signals in neoplastic transformation stems from mutational analysis of regulatory genes operational in wnt signal transduction. For example, certain mutations in β-catenin render this protein refractory to inhibition by APC and thus prevent its degradation. In consequence, constituitive activation of β-catenm/TCF regulated gene transcription occurs. Similarly, mutational changes in APC and axin can disrupt the normal regulation of β-catenin and have been associated with various forms of tumours. Thus, characterizing wnt molecules, their signal transduction pathway and their biological effects will further aid the interpretation of direct and epigenetic evidence implicating wnts in oncogenesis including the generation of colorectal carcinoma, familial adenomatous polyposis, sporadic desmoid (i.e. aggressive fibromatosis), gastric cancer, hepatoblastoma, Wilm's tumor, melanoma, pancreatic tumors, anaplastic thyroid tumors, medulloblastoma, endometrial ovarian cancer, prostate cancer, and acute lymphoblastic leukemia.

### Brief Description of the Figures

Figure 1 is a schematic picture of the Wnt signaling pathway. The abreviations used are:
   sFRP : sluable Frizzled Receptor Protein
   Fz: Frizzled
   Dvl: Dishevelled
   JNK: Jun-kinase pathway
   PI3-K: Phosphatidylinositide 3 OH Kinase
   Akt: Protein Kinase B
   GSK-3β: Glycogen Synthase Kinase 3β
   TCF: T Cell Factor
Figure 2 is a comparison of the N-terminal amino acid sequences of mouse, human and zebra fish Shoca proteins.
Figure 3 is a comparison of the SH2 domain of mouse and human Shoca-1 and Shoca-2.
Figure 4 is an alignment of the amino acid sequences of mouse and human Shoca-1.

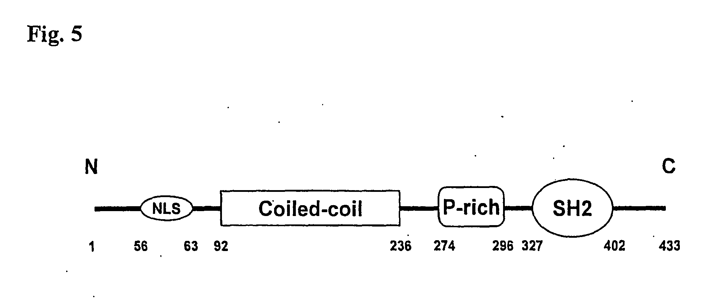

Figure 5 shows a structure prediction of Shoca-1, revealing a coiled-coiled domain and a C-terminally located SH2 domain.
Figure 6 shows Shoca-1 (Figure 6A) and Shoca-2 (Figure 6B) expression profiles in adult C57B6 mouse tissues analysed using a TaqMan assay.
Figure 7 shows the results of a luciferase reporter gene assay demonstrating that Shoca-1 modulates the transcriptional activation of β-catenin-LEF/TCF regulated activation. Expression of wild-type mouse Shoca-1 suppresses the spontaneous activity of a reporter gene while a mutant (R-K) disabling the SH2 domain fails to exert a suppressive effect depending on the cellular context.

### Brief Description of the Sequences

SEQ ID NO: 1 shows the nucleotide and amino acid sequences of murine Shoca-1.
SEQ ID NO: 2 shows the amino acid sequence of murine Shoca-1.
SEQ ID NO: 3 shows the nucleotide and amino acid sequences of human Shoca-1.
SEQ ID NO: 4 shows the amino acid sequence of human Shoca-1.
SEQ ID NO: 5 shows the nucleotide and amino acid sequences of murine Shoca-2.
SEQ ID NO: 6 shows the amino acid sequence of murine Shoca-2.
SEQ ID NO: 7 shows the nucleotide and amino acid sequences of human Shoca-2.
SEQ ID NO: 8 shows the amino acid sequence of human Shoca-2.
SEQ ID NO: 9 shows the N-terminal amino acid sequence of human Shoca-1.
SEQ ID NO: 10 shows the N-terminal amino acid sequence of murine Shoca-1.
SEQ ID NO: 11 shows the N-terminal amino acid sequence of human Shoca-2.
SEQ ID NO: 12 shows the N-terminal amino acid sequence of murine Shoca-2.
SEQ ID NO: 13 shows the N-terminal amino acid sequence of zebra fish homologue A.
SEQ ID NO: 14 shows the N-terminal amino acid sequence of zebra_ fish homologue B.
SEQ ID NO: 15 shows the amino acid sequence of the SH2 domain of human Shoca-1.
SEQ ID NO: 16 shows the amino acid sequence of the SH2 domain of murine Shoca-1.
SEQ ID NO: 17 shows the amino acid sequence of the SH2 domain of human Shoca-2.
SEQ ID NO: 18 shows the amino acid sequence of the SH2 domain of murine Shoca-2.
SEQ ID NO: 19 shows the amino acid sequence of peptide Shoca #0 used to generate Shoca antibodies.
SEQ ID NO: 20 shows the amino acid sequence of peptide Shoca #1 used to generate anti-Shoca antibodies.
SEQ ID NO: 21 shows the amino acid sequence of peptide Shoca #2 used to generate anti-Shoca antibodies.
SEQ ID NO: 22 shows the amino acid sequence of peptide Shoca #3 used to generate anti-Shoca antibodies.
SEQ ID NO: 23 shows the amino acid sequence of peptide Shoca #4 used to generate anti-Shoca antibodies.
SEQ ID NO: 24 shows the amino acid sequence of peptide Shoca #5 used to generate anti-Shoca antibodies.

### Summary of the Invention

The present inventors have identified in mouse and human tissue a novel gene whose product is directly involved in wnt-mediated signalling. The gene (SEQ ID NO: 1 and SEQ ID NO: 3) encodes an SH2-domain containing adaptor protein of 52 kDa (as demonstrated by Western blotting) and has thus been designated Shoca-1. Subsequently, a second mouse Shoca-like gene (Shoca-2) has been identified by use of EST analysis in the public domain (SEQ ID NO: 5). A human Shoca-2 homologue has also been identified on the basis of sequence homology (SEQ ID NO: 7).

The inventors have carried out an extensive molecular analysis of Shoca, characterised its function *ex vivo* and have shown that its expression in normal tissues is absent or low and that its expression is correlated with oncogenic changes in various tissues. The expression pattern of Shoca-1 and the functional effects of Shoca-mediated modulation of Wnt signals suggest a central role for this family of molecules in the process of cell fate determination. In analogy to other molecules affecting Wnt signal transduction, impairment of regular Shoca function may affect the cellular homeostasis in different tissues leading from physiological cell growth and differentiation to aberrant cell survival and function. In this context, detection of a functional aberration of Shoca has a diagnostic significance in the prediction of disease progression, therapeutic response and prognosis. Moreover, novel interacting molecules that modulate Shoca function will provide unique tools to interfere with uncontrolled cell differentiation and proliferation. The inventors have thus shown that Shoca may be important in regulating normal physiology and homeostasis of cells and that Shoca may be useful for diagnosis, risk assessment and therapy of human malignancies.

Accordingly, the present invention provides:
- an isolated Shoca polypeptide comprising:
   (i) the amino acid sequence of SEQ ID NO; 2 or SEQ ID NO: 4; or
   (ii) a variant thereof which is capable of interacting with a polypeptide of the wnt signalling pathway; or
   (iii) a fragment of (i) or (ii) which is capable of interacting with a polypeptide of the wnt signalling pathway;
- a polynucleotide encoding a polypeptide according to the invention;
- a polynucleotide encoding a Shoca polypeptide capable of interacting with a polypeptide of the wnt signalling pathway, which polynucleotide comprises:
   (i) the nucleic acid sequence of SEQ ID NO: 1 or SEQ ID NO: 3 and/or a sequence complementary thereto;
   (ii) a sequence which hybridizes under stringent conditions to a sequence defined in (i);
   (iii) a sequence that is degenerate as a result of the genetic code to a sequence as defined in (i) or (ii); or
   (iv) a sequence having at least 85% identity to a sequence as defined in (i), (ii) or (iii);
- an expression vector comprising a polynucleotide according to the invention;
- a host cell comprising a vector according to the invention;
- an antibody specific for a polypeptide according to the invention;
- a method of identifying an agent capable of modulating the wnt signalling pathway, which method comprises:
   (i) providing a polypeptide comprising the amino acid sequence of SEQ ID NO: 2 or 4, or a variant thereof, or a fragment of either thereof which variant or fragment is capable of interacting with a polypeptide of the wnt signalling pathway or a polynucleotide encoding said polypeptide; and a test agent;
   (ii) contacting the polypeptide or polynucleotide and the test agent; and
   (iii) determining whether the test agent has any effect on the expression of said polypeptide or on a function or property of said polypeptide thereby determining whether the test agent is capable of modulating Shoca activity;
- an agent capable of modulating Shoca activity identified by a method according to the invention for use in a method of treatment of the human or animal body by therapy or in a method of diagnosis carried out on the human or animal body;
- use of an agent identified by a method according to the invention in the manufacture of a diagnostic agent or a medicament for use in the diagnosis or treatment of cancer;
- a method of diagnosing cancer which method comprises determining the level of Shoca expression in a tissue sample from a subject;
- a method of predicting the progression of a tumour which method comprises determining the level of Shoca_expression in a tissue sample from a subject;
- use of an anticancer agent in the manufacture of a medicament for treating cancer in an individual wherein the individual has been diagnosed as having cancer using a method according to the invention; and
- a method of treating cancer, which method comprises:
   (i) identifying an agent capable of modulating Shoca activity; and
   (ii) administering a therapeutically effective amount of said agent to a human or animal subject in need to thereof.

### Detailed Description of the Invention

### Proteins

The present invention relates to a novel protein of the wnt signaling pathway, referred to herein as Shoca, functional variants thereof and functional fragments of Shoca or of variants of Shoca. Sequence information for murine Shoca-1 is provided in SEQ ID NO: 1 (nucleotide and amino acid) and in SEQ ID NO: 2, sequence information for human Shoca-1 is provided in SEQ ID NO: 3 (nucleotide and amino acid) and in SEQ ID NO: 4, sequence information for murine Shoca-2 is provided in SEQ ID NO: 5 (nucleotide and amino acid) and in SEQ ID NO: 6 and sequence information for human Shoca-2 is provided in SEQ ID NO: 7 (nucleotide and amino acid) and in SEQ ID NO: 8. A polypeptide of the invention thus consists essentially of the amino acid sequence of SEQ ID NO: 2, 4, 6 or 8 or of a variant of any one of these sequences, or of a fragment of any one of these sequences or variants.

Polypeptides of the invention may be in a substantially isolated form. It Will, be understood that the polypeptide may be mixed with carriers or diluents which will not interfere with the intended purpose of the polypeptide and still be regarded as substantially isolated. A polypeptide of the invention may also be in a substantially purified form, in which case it will generally comprise the polypeptide in a preparation in which more than 50%, e.g. more than 80%, 90%, 95% or 99%, by weight of the polypeptide in the preparation is a polypeptide of the invention. Routine methods can be employed to purify and/or synthesise the proteins according to the invention. Such methods are well understood by persons skilled in the art, and include techniques such as those disclosed in Sambrook et al, Molecular Cloning: a Laboratory Manual, 2nd Edition, CSH Laboratory Press, 1989, the disclosure of which is included herein in its entirety by way of reference.

The term "variant" refers to a polypeptide which shares at least one property or function with Shoca-1. A "fragment" of the invention also possesses at least one function or property of Shoca-1. Shoca-1 is a signalling protein of the wnt signaling pathway. Shoca-1 may also be a signalling protein of any other pathway regulating cell survival, proliferation or differentiation. Preferably a variant polypeptide is one which is capable of interacting with a molecule of the wnt signalling pathway, preferably a nuclear molecule. Preferably, a variant polypeptide is capable of interacting with LEF, TCF and/or β-catenin. A variant may interact with an endogenous Shoca protein. Preferably the variant polypeptide is capable of modulating β-catenin-LEF/TCF regulated transcription. Preferably modulation of β-catenin-LEF/TCF transcription by a polypeptide of the invention is tissue specific, for example with repression occurring in thymic epithelial cells and stimulation occurring in fibroblasts.

A variant polypeptide of the invention may typically be identified by monitoring for a function of Shoca such as binding to LEF and/or TCF and/or β-catenin, modulating expression of a reporter gene under the control of LEF/TCF responsive control sequences.

The SH2 domain of Shoca-1 is essential for the repression of LEF/TCF mediated transcription in thymic epithelial cells. It is, therefore, preferred that a variant or fragment of Shoca comprises a functional SH2 domain. Other preferred fragments and variants and variants may comprise other functional domains of Shoca-1, such as a coiled-coil domain or a phosphorylation site.

Shoca is almost exclusively expressed in the nucleus. Preferred fragments and variants may contain a nuclear localisation sequence. Mutants of Shoca-1 missing the first N-terminal 50 amino acids fail to translocate from the cytoplasm to the nucleus, suggesting that a motif within this sequence contains a nuclear localisation sequence. It is, therefore, preferred that a variant or fragment of the invention comprises an N-terminal domain including a nuclear localisation sequence.

More preferably, the variant or fragment contains the sequence from position 56 to position 63 of SEQ ID NO: 4.

In another aspect of the invention, a variant is one which does not show the same activity as Shoca but is one which inhibits a basic function of Shoca, i.e. has dominant-negative activity. For example, a variant polypeptide is one which inhibits modulation of LEF/TCF mediated transcription by Shoca.

The identity between the different Shoca proteins at the amino acid level are as follows: Human Shoca-1 is 91% identical to mouse Shoca-1; human Shoca-2 is 72% identical to mouse Shoca-2; human Shoca-1 is 40% identical to human Shoca-2; mouse Shoca-1 is 40% identical to mouse Shoca-2.

Typically, polypeptides with more than about 40% identity preferably at least 70%, at least 80% or at least 90% and particularly preferably at least 91 % at least 95% at least 97% or at least 99% identity, with the amino acid sequence of SEQ ID NO: 2 or 4, are considered as variants of the proteins. Such variants may include allelic variants and the deletion, modification or addition of single amino acids or groups of amino acids within the protein sequence, as long as the peptide retains at least one function or property of Shoca. Preferably a variant of SEQ I NO: 2 or 4 will have the same domain structure as Shoca-1, i.e. a coiled-coil domain and/or a C-terminal SH2 domain.

Amino acid substitutions may be made, for example from 1, 2 or 3 to 10, 20 or 30 substitutions. The modified polypeptide generally retains activity as a wnt signaling molecule. Conservative substitutions may be made, for example according to the following Table. Amino acids in the same block in the second column and preferably in the same line in the third column may be substituted for each other.

| | | |
|---|---|---|
| ALIPHATIC | Non-polar | GA P I L V |
| | Polar-uncharged | C S T M |
| | | N Q |
| | Polar-charged | D E |
| | | K R |
| AROMATIC | | H F W Y |

Variant polypeptides within the scope of the invention may be generated by any suitable method, for example by gene shuffling (molecular breeding) techniques.

Shorter polypeptide sequences are within the scope of the invention. For example, a peptide fragment of at least 20 amino acids or up to 50, 60, 70, 80, 100, 150 or 200 amino acids in length is considered to fall within the scope of the invention as long as it demonstrates a basic biological functionality of Shoca. In particular, but not exclusively, this aspect of the invention encompasses the situation when the protein is a fragment of the complete protein sequence and may represent a LEF/TCF-binding region. Such fragments can be used to construct chimeric molecules. Such fragments of Shoca or a variant thereof can also be used to raise anti-Shoca antibodies.

WO 01/54733 identifies (amongst many others) an amino acid sequence of 235 amino acids in length corresponding to residue 220 through to 454 of the amino acid sequence of SEQ ID NO: 7. No function is attributed to this peptide in WO 01/54733. This peptide is not a preferred fragment in accordance with the present invention. WO 01/53455 identifies, amongst many others, SEQ ID NO: 931, the sequence of which, beginning at the fourth residue, corresponds to residues 266 through to 454 of the amino acid sequence of SEQ ID NO: 7. No specific function is attributed to this peptide in WO 01/53455. This peptide is not a preferred fragment in accordance with the present invention.

Polypeptides of the invention may be chemically modified, e.g. post-translationally modified. For example, they may be glycosylated or comprise modified amino acid residues. They may also be modified by the addition of histidine residues to assist their purification or by the addition of a nuclear localisation sequence to promote translocation to the nucleus. Such modified polypeptides fall within the scope of the term "polypeptide" of the invention.

### Polynucleotides

The invention also includes nucleotide sequences that encode for Shoca or a variant or fragment thereof as well as nucleotide sequences which are complementary thereto. The nucleotide sequence may be RNA or DNA including genomic DNA, synthetic DNA or cDNA. Preferably the nucleotide sequence is a DNA sequence and most preferably, a cDNA sequence. Nucleotide sequence information for murine and human Shoca-1 is provided in SEQ ID NOs: 1 and 3 respectively and nucleotide sequence information for murine and human Shoca-2 is provided in SEQ ID NOs: 5 and 7 respectively. Such nucleotides can be isolated from cells or synthesised according to methods well known in the art, as described by way of example in Sambrook *et al,* 1989.

Typically a polynucleotide of the invention comprises a contiguous sequence of nucleotides which is capable of hybridizing under selective conditions to the coding sequence or the complement of the coding sequence of SEQ ID NO: 1. Such sequences include the sequences shown in SEQ ID NOs: 3, 5 and 7.

A polynucleotide of the invention can hybridize to the coding sequence or the complement of the coding sequence of SEQ ID NO: 1 at a level significantly above background. Background hybridization may occur, for example, because of other cDNAs present in a cDNA library. The signal level generated by the interaction between a polynucleotide of the invention and the coding sequence or complement of the coding sequence of SEQ ID NO: 1 is typically at least 10 fold, preferably at least 100 fold, as intense as interactions between other polynucleotides and the coding sequence of SEQ ID NO: 1. The intensity of interaction may be measured, for example, by radiolabelling the probe, e.g. with ³²P. Selective hybridisation may typically be achieved using conditions of medium to high stringency. However, such hybridisation may be carried out under any suitable conditions known in the art (see Sambrook *et al,* 1989. For example, if high stringency is required suitable conditions include from 0.1 to 0.2 x SSC at 60 °C up to 65 °C. If lower stringency is required suitable conditions include 2 x SSC at 60 °C.

The coding sequence of SEQ ID NO: 1, 3, 5 or 7 may be modified by nucleotide substitutions, for example from 1, 2 or 3 to 10, 25, 50 or 100 substitutions. The polynucleotide of SEQ ID NO: 1, 3, 5 or 7 may alternatively or additionally be modified by one or more insertions and/or deletions and/or by an extension at.either or both ends. A polynucleotide may include one or more introns, for example may comprise genomic DNA. The modified polynucleotide generally encodes a polypeptide which has Shoca activity. Alternatively, a polynucleotide encodes a ligand-binding portion of a polypeptide or a polypeptide which modulates Shoca activity. Degenerate substitutions may be made and/or substitutions may be made which would result in a conservative amino acid substitution when the modified sequence is translated, for example as shown in the Table above.

The identity between the different Shoca proteins at the DNA level are as follows: Human Shoca-1 is 80% identical to mouse Shoca-1; human Shoca-2 is 75% identical to mouse Shoca-2, human Shoca-1 is 57% identical to human Shoca-2; and mouse Shoca-1 is 59% identical to mouse Shoca-2.

A nucleotide sequence which is capable of selectively hybridizing to the complement of the DNA coding sequence of SEQ ID NO: 1 or 3 will generally have at least 50%, at least 57%, at least 60%, at least 70%, at least 80%, at least 88%, at least 90%, at least 95%, at least 98% or at least 99% sequence identity to the coding sequence of SEQ ID NO: 1 over a region of at least 20, preferably at least 30, for instance at least 40, at least 60, more preferably at least 100 contiguous nucleotides or most preferably over the full length of SEQ ID NO: 1. Preferably the nucleotide sequence encodes a polypeptide which has the same domain structure as Shoca-1, i.e. a coiled-coil domain and/or a C-terminal SH2 domain.

For example the UWGCG Package provides the BESTFIT program which can be used to calculate homology (for example used on its default settings) (Devereux et al (1984) Nucleic Acids Research 12, p387-395). The PILEUP and BLAST algorithms can be used to calculate homology or line up sequences (typically on their default settings), for example as described in Altschul (1993) J. Mol. Evol. 36:290-300; Altschul et al (1990) J. Mol. Biol. 215:403-10.

Software for performing BLAST analyses is publicly available through the National Centre for Biotechnology Information (http://www.ncbi.nlm.ni.h.govn/). This algorithm involves first identifying high scoring sequence pair (HSPs) by identifying short words of length W in the query sequence that either match or satisfy some positive-valued threshold score T when aligned with a word of the same length in a database sequence. T is referred to as the neighbourhood word score threshold (Altschul *et al;* 1990). These initial neighbourhood word hits act as seeds for initiating searches to find HSPs containing them. The word hits are extended in both directions along each sequence for as far as the cumulative alignment score can be increased. Extensions for the word hits in each direction are halted when: the cumulative alignment score falls off by the quantity X from its maximum achieved value; the cumulative score goes to zero or below, due to the accumulation of one or more negative-scoring residue alignments; or the end of either sequence is reached. The BLAST algorithm parameters W, T and X determine the sensitivity and speed of the alignment. The BLAST program uses as defaults a word length (W) of 11, the BLOSUM62 scoring matrix (see Henikoff and Henikoff (1992) Proc. Natl. Acad. Sci. USA 89: 10915-10919) alignments (B) of 50, expectation (E) of 10, M=5, N=4, and a comparison of both strands.

The BLAST algorithm performs a statistical analysis of the similarity between two sequences; see e.g., Karlin and Altschul (1993) Proc. Natl. Acad. Sci. USA 90: 5873-5787 and Altschul and Gish (1996) Methods Enzymol. 266: 460-480. One measure of similarity provided by the BLAST algorithm is the smallest sum probability (P(N)), which provides an indication of the probability by which a match between two nucleotide or amino acid sequences would occur by chance. For example, a sequence is considered similar to another sequence if the smallest sum probability in comparison of the first sequence to the second sequence is less than about 1, preferably less than about 0.1, more preferably less than about 0.01, and most preferably less than about 0.001.

Any combination of the above mentioned degrees of sequence identity and minimum sizes may be used to define polynucleotides of the invention, with the more stringent combinations (i.e. higher sequence identity over longer lengths) being preferred. Thus, for example a polynucleotide which has at least 90% sequence identity over 25, preferably over 30 nucleotides forms one aspect of the invention, as does a polynucleotide which has at least 95% sequence identity over 40 nucleotides.

The nucleotides according to the invention have utility in production of the proteins according to the invention, which may take place *in vitro, in vivo* or *ex vivo.* The nucleotides may be involved in recombinant protein synthesis or indeed as therapeutic agents in their own right, utilised in gene therapy techniques. Nucleotides complementary to those encoding Shoca, or antisense sequences, may also be used in gene therapy.

The present invention also includes expression vectors that comprise nucleotide sequences encoding the proteins of the invention. Such expression vectors are routinely constructed in the art of molecular biology and may for example involve the use of plasmid DNA and appropriate initiators, promoters, enhancers and other elements, such as for example polyadenylation signals which may be necessary; and which are positioned in the correct orientation, in order to allow for protein expression. Other suitable vectors would be apparent to persons skilled in the art. By way of further example in this regard we refer to Sambrook *et al.* 1989.

Flanking sequences upstream from the ATG site in the Shoca gene are important for the regulation of Shoca expression. It is preferred that flanking sequences needed for the proper expression of Shoca are included in expression vectors of the invention.

Polynucleotides according to the invention may also be inserted into the vectors described above in an antisense orientation in order to provide for the production of antisense RNA. Antisense RNA or other antisense polynucleotides may also be produced by synthetic means. Such antisense polynucleotides may be used as test compounds in the assays of the invention or may be useful in a method of treatment of the human or animal body by therapy.

Preferably, a polynucleotide of the invention in a vector is operably linked to a control sequence which is capable of providing for the expression of the coding sequence by the host cell, i.e. the vector is an expression vector. The term "operably linked" refers to a juxtaposition wherein the components described are in a relationship permitting them to function in their intended manner. A regulatory sequence, such as a promoter, "operably linked" to a coding sequence is positioned in such a way that expression of the coding sequence is achieved under conditions compatible with the regulatory sequence.

The vectors may be for example, plasmid, virus or phage vectors provided with a origin of replication, optionally a promoter for the expression of the said polynucleotide and optionally a regulator of the promoter. The vectors may contain one or more selectable marker genes, for example an ampicillin resistance gene in the case of a bacterial plasmid or a resistance gene for a fungal vector. Vectors may be used *in vitro,* for example for the production of DNA or RNA or used to transfect or transform a host cell, for example, a mammalian host cell. The vectors may also be adapted to be used *in vivo,* for example in a method of gene therapy.

Promoters and other expression regulation signals may be selected to be compatible with the host cell for which expression is designed. For example, yeast promoters include *S*. *cerevisiae* GAL4 and ADH promoters, *S. pombe nmt*1 and *adh* promoter. Mammalian promoters include the metallothionein promoter which can be induced in response to heavy metals such as cadmium. Viral promoters such as the SV40 large T antigen promoter or adenovirus promoters may also be used. An IRES promoter may also be used. All these promoters are readily available in the art.

Mammalian promoters, such as β-actin promoters, may be used. Tissue-specific promoters are especially preferred. Viral promoters may also be used, for example the Moloney murine leukaemia virus long terminal repeat (MMLV LTR), the rous sarcoma virus (RSV) LTR promoter, the SV40 promoter, the human cytomegalovirus (CMV) IE promoter, adenovirus, HSV promoters (such as the HSV IE promoters), or HPV promoters, particularly the HPV upstream regulatory region (URR). Viral promoters are readily available in the art.

The vector may further include sequences flanking the polynucleotide giving rise to polynucleotides which comprise sequences homologous to eukaryotic genomic sequences, preferably mammalian genomic sequences, or viral genomic sequences. This will allow the introduction of the polynucleotides of the invention into the genome of eukaryotic cells or viruses by homologous recombination. In particular, a plasmid vector comprising the expression cassette flanked by viral sequences can be used to prepare a viral vector suitable for delivering the polynucleotides of the invention to a mammalian cell. Other examples of suitable viral vectors include herpes simplex viral vectors and retroviruses, including lentiviruses, adenoviruses, adeno-associated viruses and HPV viruses. Gene transfer techniques using these viruses are known to those skilled in the art. Retrovirus vectors for example may be used to stably integrate the polynucleotide giving rise to the polynucleotide into the host genome. Replication-defective adenovirus vectors by contrast remain episomal and therefore allow transient expression.

The invention also includes cells that have been modified to express a Shoca polypeptide of the invention. Such cells include transient, or preferably stable higher eukaryotic cell lines, such as mammalian cells or insect cells, using for example a baculovirus expression system, lower eukaryotic cells, such as yeast or prokaryotic cells such as bacterial cells. Particular examples of cells which may be modified by insertion of vectors encoding for a polypeptide according to the invention include mammalian thymic epithelial cells, fibroblasts, HEK293T, CHO, HeLa, BHK, 3T3 and COS cells. A polypeptide of the invention may be expressed in cells of a transgenic non-human animal, preferably a mouse. A transgenic non-human animal expressing a polypeptide of the invention is included within the scope of the invention.

### Antibodies

According to another aspect, the present invention also relates to antibodies, specific for a polypeptide of the invention. Such antibodies are for example useful in purification, isolation or screening methods involving immunoprecipitation techniques or, indeed, as therapeutic agents in their own right. Antibodies may be raised against specific epitopes of the polypeptides according to the invention.

Preferred antibodies are raised against the amino acid sequences shown in SEQ ID Nos: 19, 20 and 21.

Antibodies may be used to impair Shoca function. An antibody, or other compound, "specifically binds" to a protein when it binds with preferential or high affinity to the protein for which it is specific but does substantially bind not bind or binds with only low affinity to other proteins. A variety of protocols for competitive binding or immunoradiometric assays to determine the specific binding capability of an antibody are well known in the art (see for example Maddox et al, J. Exp. Med. 158,1211-1226,1993). Such immunoassays typically involve the formation of complexes between the specific protein and its antibody and the measurement of complex formation.

Antibodies of the invention may be antibodies to human polypeptides or fragments thereof. For the purposes of this invention, the term "antibody", unless specified to the contrary, includes fragments which bind a polypeptide of the invention. Such fragments include Fv, F(ab') and F(ab')₂ fragments, as well as single chain antibodies. Furthermore, the antibodies and fragment thereof may be chimeric antibodies, CDR-grafted antibodies or humanised antibodies.

Antibodies may be used in a method for detecting polypeptides of the invention in a biological sample, which method comprises:
I providing an antibody of the invention;
II incubating a biological sample with said antibody under conditions which allow for the formation of an antibody-antigen complex; and
III determining whether antibody-antigen complex comprising said antibody is formed.

A sample may be for example a tissue extract, blood, serum and saliva. Antibodies of the invention may be bound to a solid support and/or packaged into kits in a suitable container along with suitable reagents, controls, instructions, etc. Antibodies may be linked to a revealing label and thus may be suitable for use in methods of *in vivo* Shoca imaging.

Antibodies of the invention can be produced by any suitable method. Means for preparing and characterising antibodies are well known in the art, see for example Harlow and Lane (1988) "Antibodies: A Laboratory Manual", Cold Spring Harbor Laboratory Press, Cold Spring Harbor, NY. For example, an antibody may be produced by raising antibody in a host animal against the whole polypeptide or a fragment thereof, for example an antigenic epitope thereof, herein after the "immunogen".

A method for producing a polyclonal antibody comprises immunising a suitable host animal, for example an experimental animal, with the immunogen and isolating immunoglobulins from the animal's serum. The animal may therefore be inoculated with the immunogen, blood subsequently removed from the animal and the IgG fraction purified.

A method for producing a monoclonal antibody comprises immortalising cells which produce the desired antibody. Hybridoma cells may be produced by fusing spleen cells from an inoculated experimental animal with tumour cells (Kohler and Milstein (1975) Nature 256, 495-497).

An immortalized cell producing the desired antibody may be selected by a conventional procedure. The hybridomas may be grown in culture or injected intraperitoneally for formation of ascites fluid or into the blood stream of an allogenic host or immunocompromised host. Human antibody may be prepared by *in vitro* immunisation of human lymphocytes, followed by transformation of the lymphocytes with Epstein-Barr virus and in transgenic mice enabling production of human antibodies.

For the production of both monoclonal and polyclonal antibodies, the experimental animal is suitably a goat, rabbit, rat or mouse. If desired, the immunogen may be administered as a conjugate in which the immunogen is coupled, for example via a side chain of one of the amino acid residues, to a suitable carrier. The carrier molecule is typically a physiologically acceptable carrier. The antibody obtained may be isolated and, if desired, purified.

### Assays

An important aspect of the present invention is the use of polypeptides according to the invention in screening methods. The screening methods may be used to identify substances that bind to Shoca polypeptides or mRNAs. Screening methods may also be used to identify modulators, which may be inhibitors or activators of Shoca activity, and/or agents which up-regulate or down-regulate Shoca expression. Generally, an agent capable of binding Shoca, modulating Shoca activity and/or modulating Shoca expression will be capable of modulating the wnt signalling pathway.

Any suitable format may be used for the assay. In general terms such screening methods may involve contacting a polypeptide of the invention with a test agent and monitoring for binding of the test agent to the polypeptide or measuring Shoca activity. A polypeptide of the invention may be incubated with a test agent. Modulation of Shoca activity may be determined. In a preferred aspect, the assay is a cell-based assay. Preferably the assay may be carried out in a single well of a microtitre plate. Assay formats which allow high throughput screening are preferred.

Modulator activity can be determined by contacting cells expressing a polypeptide of the invention with an agent under investigation and by monitoring an effect mediated by the polypeptide. The cells expressing the polypeptide may be *in vitro* or *in vivo*. The polypeptide of the invention may be naturally or recombinantly expressed. Preferably, the assay is carried out *in vitro* using cells expressing recombinant polypeptide. Preferably, control experiments are carried out on cells which do not express the polypeptide of the invention to establish whether the observed responses are the result of activation of the polypeptide. Typically the cells will express other molecules of the wnt signalling pathway such as β-catenin, LEF and /or TCF.

A method of identifying an agent capable of modulating the wnt signalling pathway, may consist essentially of:
(i) providing a polypeptide of the invention or a polynucleotide of the invention encoding said polypeptide and a test agent;
(ii) contacting the polypeptide or polynucleotide and the test agent; and
(iii) monitoring any interaction between the polypeptide or polynucleotide and the test agent, thereby determining whether the test agent is capable of modulating the Wnt signalling pathway.

An interaction between the polypeptide or polynucleotide and the test agent may be monitored directly by monitoring binding of the polypeptide or polynucleotide to the test agent. Preferably direct binding of the test agent to the polypeptide or to the mRNA encoding the polypeptide is monitored. For example, a radiolabelled test agent can be incubated with the polypeptide of the invention and binding of the test agent to the polypeptide can be monitored.

Assays may be carried out using cells expressing Shoca, and incubating such - cells with the test agent. The results of the assay are compared to the results obtained using the same assay in the absence of the test agent. Cells expressing Shoca constitutively may be provided for use in assays for Shoca function. Alternatively, an interaction between the polypeptide or polynucleotide and the test agent may be monitored indirectly by monitoring activity of a Shoca polypeptide of the invention. An agent capable of modulating the wnt signalling pathway may be an inhibitor of Shoca activity or may be an activator of Shoca activity.

Shoca activity may be determined by monitoring an effect of stimulation or inhibition of Shoca activity on cells, for example by monitoring cell proliferation, differentiation, growth or survival.

Shoca activity may be determined by monitoring phosphorylation of a polypeptide of the invention and determining whether the test agent inhibits or enhances phosphorylation.

Shoca binds directly or indirectly to LEF/TCF transcription factors and/or co-associates with β-catenin in the nucleus. Shoca may repress or activate β-catenin-LEF/TCF mediated transcription in the nucleus depending on the cellular context. For Shoca to exert an effect on such transcription it must be present in the nucleus. Therefore, Shoca activity may be determined by monitoring the intracellular location of a polypeptide of the invention, and in particular translocation to the nucleus.

A method of the invention may be used to identify an agent which inhibits or enhances binding of Shoca to a molecule of the wnt signalling pathway such as β-catenin, LEF and/or TCF. A method of identifying an agent capable of modulating the wnt signalling pathway according to the invention may further comprise providing a molecule of the wnt signalling pathway capable of interacting with Shoca. The polypeptide or polynucleotide and the test agent and the wnt signalling molecule may then be contacted under conditions suitable for the interaction of the polypeptide and the wnt signalling molecule and the effect of the test agent on the interaction of the polypeptide and the wnt signalling molecule may be determined by monitoring the interaction. Preferably the wnt signalling molecule is β-catenin, TCF, LEF or any combination of β-catenin, TCF or/and LEF.

Substances that inhibit the interaction of a Shoca polypeptide of the invention with a wnt signalling molecule such as β-catenin, LEF or TCF may also be identified through a mammalian 2-hybrid assay, yeast 2-hybrid assay, yeast 3-hybrid assay (protein-DNA interaction assay) or other protein interaction assay such as a co-immunoprecipitation or an ELISA based technique.

Agents capable of modulating the wnt signalling pathway may be identified by determining whether a test agent inhibits or enhances modulation of gene transcription by a Shoca polypeptide of the invention. The effect of a test agent on Shoca-mediated repression or activation of gene expression may thus be monitored in a method of the invention. Typically, a reporter gene construct comprising a reporter gene under the transcriptional control of LEF and/or TCF is provided and contacted with a polypeptide of the invention and a test agent under conditions suitable for expression of a reporter gene. Expression of the reporter gene may be monitored and compared to expression in a control experiment without the test agent to determine whether the test agent modulates Shoca activity.

Assays may also be carried out to identify agents which modify Shoca expression, for example substances which up- or down- regulate expression. Generally, in such assays a polynucleotide of the invention is contacted with the test agent. The polynucleotide may be mRNA and the test agent may modulate translation. Preferably the polynucleotide is DNA and the test agent preferably modulates transcription. Such assays may, alternatively, be carried out for example by using antibodies for Shoca to monitor levels of Shoca expression.

Additional control experiments may be carried out.

Suitable test agents which can be tested in the above assays include combinatorial libraries, defined chemical entities and compounds, peptide and peptide mimetics, oligonucleotides and natural product libraries, such as display (e.g. phage display libraries) and antibody products.

Typically, organic molecules will be screened, preferably small organic molecules which have a molecular weight of from 50 to 2500 daltons. Candidate products can be biomolecules including, saccharides, fatty acids, steroids, purines, pyrimidines, derivatives, structural analogs or combinations thereof. Candidate agents are obtained from a wide variety of sources including libraries of synthetic or natural compounds. Known pharmacological agents may be subjected to directed or random chemical modifications, such as acylation, alkylation, esterification, amidification, etc. to produce structural analogs.

Test agents may be used in an initial screen of, for example, 10 agents per reaction, and the agents of these batches which show inhibition or activation tested individually. Test agents may be used at a concentration of from InM to 1000µM, preferably from 1µM to 100µM, more preferably from 1µM to 10µM. Preferably, Shoca activity in the presence of a test agent is compared to the activity shown in the absence of the test agent. A test agent which acts as an inhibitor may produce a 50% inhibition of Shoca activity. Alternatively a test agent which acts as an activator may enhance Shoca activity by 50%.

The activity of Shoca on β-catenin-LEF/TCF transcriptional activity is tissue-specific. For example, in thymic epihelial cells Shoca acts to repress such transcriptional activity but in fibroblasts Shoca stimulates transcription of gene sequences under the control of LEF/TCF. In one embodiment the present invention provides a method for identifying a cellular component that interacts with Shoca and which is responsible for determining the tissue-specific activity of Shoca. Such a method typically comprises any assay described herein for the identification of a modulator of the wnt signalling pathway in which the test agent is a cellular component. Suitable test agents include for example, a crude cellular extract, a fraction of a cellular extract, proteins purified from a cellular extract or a protein isolated from the cell type or tissue of interest.

Another aspect of the present invention is the use of polynucleotides encoding the Shoca polypeptides of the invention to identify mutations in Shoca genes which may be implicated in human disorders and, in particular, susceptibility to cancer. Identification of such mutations may be used to assist in diagnosis or susceptibility to such disorders and in assessing the physiology of such disorders. Polynucleotides may also be used in hybridisation studies to monitor for up- or down-regulation of Shoca expression. Polynucleotides such as SEQ ID NO: 1, SEQ ID NO: 3, SEQ ID NO: 5, SEQ ID NO: 7 or fragments thereof may be used to identify allelic variants, genomic DNA and species variants.

### Diagnosis

The present invention provides a method for detecting variation in the expressed products encoded by Shoca genes. This may comprise determining the level of Shoca expressed in cells or determining specific alterations in the expressed product. Sequences of interest for diagnostic purposes include, but are not limited to, the conserved portions as identified by sequence similarity and conservation of intron/exon structure. The diagnosis may be performed in conjunction with kindred studies to determine whether a mutation of interest co-segregates with disease phenotype in a family.

The present inventors have shown that Shoca is expressed in many different cell types and neoplastic tissues. In particular, Shoca is involved in distinct tumour types including breast cancer, colon cancer and cervical cancer. Normal tissue from a broad range of organs was found to be either negative for Shoca or to express low levels of Shoca while expression of Shoca in these tissues was associated with less malignant tumour forms. Further, cancerous progression was shown to coincide with a loss of Shoca expression in many tumours. Accordingly, Shoca is a clinically relevant prognostic marker for tumours, especially breast, colon and cervical tumours. Shoca may also be used as a diagnostic maker for diseases which involve benign tissue changes such as mastopathy, apocrine metaplasia, intraductal hyperplasia; papilloma and carcinoma.

Thus in a further embodiment, the present invention provides a method of diagnosing cancer by determining the level of Shoca expression in a tissue sample from a subject. The invention also provides a method of predicting the progression of a tumour by determining the level of Shoca expression in a tissue sample from a subject. Also provided is a method of diagnosing a disease involving benign tissue changes such as mastopathy, apocrine metaplasia, intraductal hyperplasia, papilloma and carcinoma by determining the level of Shoca expression in a tissue sample from a subject. The level of Shoca expression may be determined by monitoring the level of a polypeptide of the invention or mRNA encoding a polypeptide of the invention. Any suitable tissue sample may be used, for example a biopsy or resection. Preferably the cancer is breast, colon or cervical cancer.

The expression of a Shoca protein or mRNA may be determined using an agent that interacts with Shoca. Suitable agents may be identified using a screening assay of the invention. Preferably, the agent is capable of binding specifically to the Shoca protein. More preferably the agent is an antibody of the invention.

A method of diagnosing cancer, or of predicting the progression of a tumour, or of diagnosing a disease involving benign tissue changes such as mastopathy, apocrine metaplasia, intraductal hyperplasia, papilloma and carcinoma may comprise the steps of:
(i) identifying an agent capable of interacting with Shoca;
(ii) contacting a sample from a human or animal subject with the agent;
(iii) monitoring binding of the agent to the sample; and
(iv) determining whether the level of Shoca expression is elevated or reduced in said sample compared to the level of Shoca expression in a sample from a human or animal subject not having cancer or said disease.

Diagnostic procedures may be performed on polynucleotides isolated from an individual or alternatively, may be performed *in situ* directly upon tissue sections (fixed and/or frozen) of patient tissue obtained from biopsies or resections, such that no nucleic acid purification is necessary. Appropriate procedures are described in, for example, Nuovo, G.J., 1992, "PCR *In Situ* Hybridization: Protocols And Applications", Raven Press, NY: Such analysis techniques include, DNA or RNA blotting analyses, single stranded conformational polymorphism analyses, *in situ* hybridization assays, and polymerase chain reaction analyses. Such analyses may reveal both quantitative aspects of the expression pattern of Shoca and qualitative aspects of Shoca expression and/or composition.

Alternative diagnostic methods for the detection of Shoca nucleic acid molecules may involve their amplification, e.g. by PCR (the experimental embodiment set forth in U.S. Patent No. 4,683,202), ligase chain reaction (Barany, 1991, Proc. Natl. Acad. Sci. USA 88:189-193), self sustained sequence replication (Guatelli et al., 1990, Proc. Natl. Acad Sci. USA 87:1874-1878), transcriptional amplification system (Kwoh et al., 1989, Proc. Natl. Acad. Sci. 15 USA 86:1173-1177), Q-Beta Replicase (Lizardi et al., 1988, Bio/Technology 6:1197) or any other nucleic acid amplification method (for example, Holland et al., 1991, Proc. Natl. Acad. Sci. USA 88:7276-7280), followed by the detection of the amplified molecules using techniques well known to those of skill in the art. These detection schemes are especially useful for the detection of nucleic acid molecules if such molecules are present in very low numbers.

Particularly suitable diagnostic methods are chip-based DNA technologies such as those described by Hacia et al.,1996, Nature Genetics 14:441-447, Shoemaker et al., 1996, Nature Genetics 14:450-456 and Welford et al., 1998, Nucl. Ac. Res. 26: 3059-3065. Briefly, these techniques involve quantitative methods for analyzing large numbers of nucleic acid sequence targets rapidly and accurately. By tagging with oligonucleotides or using fixed probe arrays, one can employ chip technology to segregate target molecules as high density arrays and screen these molecules on the basis of hybridization.

Following detection, the results seen in a given patient may be compared with a statistically significant reference group of normal patients and patients that have cancer. In this way, it is possible to correlate the amount or kind of Shoca encoded product detected with various cancers or predisposition to various cancers. Generally, only low levels of Shoca expression or no Shoca expression is seen in tissue from individuals not having a tumour so expression in such a tissue may be an indication of a cancerous tumour.

In an individual known to have a tumour, expression of Shoca may typically indicate that the tumour is not very aggressive and no expression of Shoca in the tumour cells may typically indicate that the tumour is malignant.

### Therapeutic Treatment

The present invention also provides a method of treating a cancer in an individual, the method comprising:
(i) carrying out a method of diagnosis according to the invention on a tissue sample from the individual; and
(ii) administering an anti-cancer agent to the individual.

Another aspect of the present invention is the use of the agents that have been identified by screening techniques referred to above in the treatment of disease states which are responsive to regulation of Shoca activity, such as cancers. In particular, such substances may be used in the treatment of colon, breast and cervical cancers. The treatment may be therapeutic or prophylactic.

Accordingly, the present invention provides an agent capable of modulating the wnt signalling pathway identified by a method of the invention for use in a method of treatment by the human or animal body by therapy or in a method of diagnosis carried out on the human or animal body. The use of an agent identified by a method of the invention in the manufacture of a medicament for use in the diagnosis or treatment of cancer is also provided.

A method of treating cancer according to the invention may consist essentially of the steps of:
(i) identifying an agent capable of modulating Shoca activity; and
(ii) administering a therapeutically effective amount of the agent to a human or animal subject in need thereof.

A human or animal subject in need of treatment may be identified by a method of diagnosis according to the invention.

A therapeutically effective amount of an agent is an amount which when administered to a patient with cancer, improves the condition of a patient. The condition of a patient may be improved if one or more symptom of cancer is allieviated. The agent may, for example, kill tumour cells or inhibit tumour progression.

Agents identified according to the screening methods outlined above may be formulated with standard pharmaceutically acceptable carriers and/or excipients as is routine in the pharmaceutical art. For example, a suitable agent may be dissolved in physiological saline or water for injections. The exact nature of a formulation will depend upon several factors including the particular agent to be administered and the desired route of administration. Suitable types of formulation are fully described in Remington's Pharmaceutical Sciences, Mack Publishing Company, Eastern Pennsylvania, 17^{th} Ed. 1985, the disclosure of which is included herein of its entirety by way of reference.

The agents may be administered by enteral or parenteral routes such as via oral, buccal, anal, pulmonary, intravenous, intra-arterial, intramuscular, intraperitoneal, topical or other appropriate administration routes.

A therapeutically effective amount of a modulator is administered to a patient. The dose of a modulator may be determined according to various parameters, especially according to the substance used; the age, weight and condition of the patient to be treated; the route of administration; and the required regimen. A physician will be able to determine the required route of administration and dosage for any particular patient. A typical daily dose is from about 0.1 to 50 mg per kg of body weight, according to the activity of the specific modulator, the age, weight and conditions of the subject to be treated, the type and severity of the degeneration and the frequency and route of administration. Preferably, daily dosage levels are from 5 mg to 2 g.

Nucleic acid encoding a Shoca polypeptide of the invention which inhibits or enhances Shoca activity or antisense nucleic acid may be administered to the mammal. Nucleic acid, such as RNA or DNA, and preferably, DNA, is provided in the form of a vector, such as the polynucleotides described above, which may be expressed in the cells of the mammal.

Nucleic acid administered to the mammal for gene therapy may encode a variant of Shoca with an impaired function such as a dominant negative mutant that disrupts the function of endogenous Shoca or may encode a constitutively active variant of Shoca that enhances the function of endogenous Shoca.

Nucleic acid encoding the polypeptide may be administered by any available technique. For example, the nucleic acid may be introduced by needle injection, preferably intradermally, subcutaneously or intramuscularly. Alternatively, the nucleic acid may be delivered directly across the skin using a nucleic acid delivery device such as particle-mediated gene delivery. The nucleic acid may be administered topically to the skin, or to mucosal surfaces for example by intranasal, oral, intravaginal or intrarectal administration.

Uptake of nucleic acid constructs may be enhanced by several known transfection techniques, for example those including the use of transfection agents. Examples of these agents includes cationic agents, for example, calcium phosphate and DEAE-Dextran and lipofectants, for example, lipofectam and transfectam. The dosage of the nucleic acid to be administered can be altered. Typically the nucleic acid is administered in the range of 1pg to 1mg, preferably to 1pg to 10µg nucleic acid for particle mediated gene delivery and 10µg to 1mg for other routes.

The following Examples illustrate the invention.

### Example 1. Shoca-1, a novel gene involved in Wnt-mediated signalling

We have recently identified in mouse and human tissue a novel gene whose product is directly involved in wnt-mediated signalling. The gene (SEQ ID NO: 1 and SEQ ID NO: 3) encodes an SH2-domain containing adaptor protein of 52 kDa (as demonstrated by Western blotting) and has thus been designated Shoca-1. Subsequently, a second Shoca-like gene has been identified by use of EST analysis in the public domain (SEQ ID NO: 5). Neither existence nor function of these genes have yet been reported in any species.

Mouse Shoca-2 was found by doing a blast search in the EST database using the mouse Shoca-1 cDNA sequence. Using the partial EST sequences, 5' and 3' RACE was performed in order to obtain the full coding sequence. Since then a full length cDNA has been submitted to the public databases with a sequence identical to mouse Shoca-2 (accession code AK008803). Similarly, human Shoca-2 was found by BLAST search of the human EST and the human genome databases. Human Shoca-2 is found in the EST database (accession code AK024799). The human Shoca-2 sequence is entirely taken from the EST database.

The chromosomal localisations in human are 10q22-q23.1 for human Shoca-1 and 8pter-p23.3 for human Shoca-2. In addition, a fragmented DNA sequence corresponding to the N-terminal part of mouse Shoca-1 was found in the public domain. However, an error in the open reading frame of this sequence introduced a translation error. By correcting this error (one base pair) an amino acid sequence of 214 residues could correctly be translated.

There is a splice variant for mouse Shoca-1 which has an alternative exon 1 and hence a different amino acid sequence at the N-terminus of the protein. So far no evidence that this variant exists in human (based on the genomic sequence in the Shoca-1 region) has been determined and it is not known if it has any biological function. The mouse and human Shoca-2 differ by a stretch of a few amino acid residues which seem to be coded in a single exon (based on the human genomic sequence). This sequence has some interesting Prosite motifs. So far it is not know if this exon is included in certain mouse transcripts or if it is lacking in certain human transcripts.

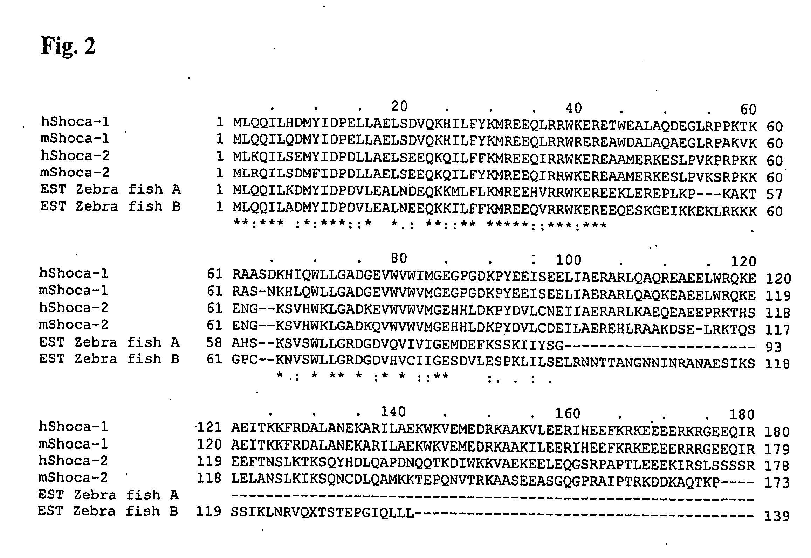

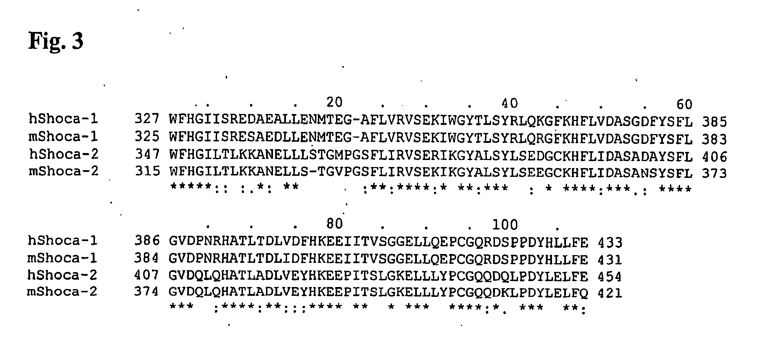

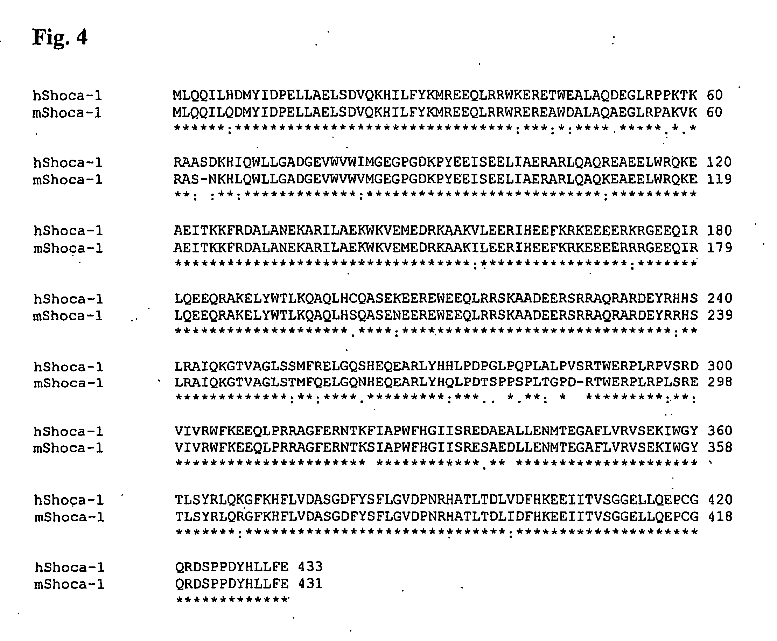

The Shoca family is well conserved between mouse and man over the entire sequence revealing a sequence homology between mouse and man of approximately 90% for Shoca-1 and 70% for Shoca-2. The amino acid sequence between the orthologs of mouse and humans displays a 91 % homology and 95% similarity (Figure 4). We have also detected in zebra fish *(D. rerio)* two Shoca family members (for which there are presently only EST sequences known with accession codes AW419549 and BE016614, respectively). The N-terminal and the C-terminal sequences, where available, of mouse, man and zebra fish are strikingly similar, thus revealing well conserved domains of possibly distinct function(s) (Figures 2 and 3). Although bearing an SH2 motif, Shoca-1 is almost exclusively expressed in the nucleus. Mutants of Shoca-1 missing the first N-terminal 50 amino-acids fail to translocate from the cytoplasm to the nucleus, suggesting that a motif within this sequence contains the information for nuclear localization. Predictions have revealed that position 56-63 (PPKTKRAA) to over 30% probability contains the NLS in human Shoca-1.

### Example 2. Generation of Shoca specific antibodies

### Antibody preparation A

The anti Shoca-1 antibodies (rabbit polyclonal serum) used to determine the subcellular localization were generated in our Institute. Different batches were used whereby the animals were bled at different times after repetitive boosting. The immunogen was a mouse Shoca-1 derived peptide coupled to the carrier protein KLH
Peptide Shoca#0: NH₂-CLPDTSPPSPLTGPDRTWERPLRC-CONH₂

This peptide represents a stretch of 22 amino acids corresponding to the mouse Shoca-1 residue positions 272 through 293. The N-terminal and C-terminal cysteines were introduced to allow loop formation on the protein carrier in order to enhance immunization.

### Antibody preparation B

The anti Shoca-1 antibodies that have been used in the staining experiments in Example 5 were generated in two rabbits by Eurogentec and also represent polyclonal preparations. All preparations used in stainings were affinity-purified using a peptide loaded column. The serum purified was derived from a-single animal following the fourth booster immunization. The immunogens represent a mixture of two mouse Shoca-1 derived peptides coupled to the carrier protein KLH and were injected concommitently:
Peptide Shoca#1: NHCOCH₃-CGEGPGDKPYEEISEEC-COOH
Peptide Shoca#2: NHCOCH₃-ADEERSRRAQRARDEYRRC-CONH₂

Peptide#1 represents stretch 83 through 97 of mouse Shoca-1 protein sequence, 15 amino acid residues and peptide#2 represents stretch 220 through 237 of mouse Shoca-1 protein sequence, 18 amino acid residues. The N-terminal (for peptide#1) and C-terminal (for both peptides) cysteines were introduced to allow loop formation on the protein carrier for immunization (peptide #1) or to introduce a C-terminal anchoring residue in peptide #2. Both peptides were coupled onto carrier proteins and injected into rabbits concomitantly.

### Antibody preparation C

The human specific anti Shoca-1 antibodies were generated in rabbits by Eurogentec and do also represent a polyclonal preparation. The immunogen represents a human Shoca-1 derived peptide coupled to the carrier protein KLH taking advantage of cysteines added to the specific Shoca sequence N- or C-terminally and the heterobifunctional crosslinker MBS. Peptide-KLH conjugates were injected subcutaneously:
Peptide Shoca#3: NHCOCH₃-CGLRPPKTKRAASDKHIQC-COOH
Peptide#3 represents the 17 amino acid residues from position 53 through 69 of the human Shoca-1 protein sequence. The preparation used in staining experiments was affinity-purified using a peptide-loaded column as described below. The purified serum was derived from a single animal following the fourth booster immunisation.

Crude antisera were filtered through 0.45 µm filters and affinity-purified on peptide columns. The free peptide, with which animals were immunised, was covalently bound to iodoacetyl-coupled crosslinked agarose via its free sulfhydryl groups (SulfoLink Coupling Gel, Pierce #20401). Unbound reactive iodacetyl groups were quenched with cysteine. Antisera of the respective reactivity were allowed to bind to the peptide-columns. The column-bound fraction was eluted with 0.1 M Glycine, pH 3.0. The eluate was immediately neutralised with 1 M Tris-HCl to achieve pH 7.0. The purified antibodies were equilibrated against PBS (pH 7.3) and concentrated in 50 kD MWCO columns (Vivascience #VS0131). The protein concentration was determined using the BCA method (Pierce #23223/23224) using purchased rabbit IgG as concentration standards (Peprotech #500-P00). The final preparation was stabilised by adding 0.02% NaN3. The serum purified derived from a single animal following the fourth booster immunisation.

### Antibody preparation D

The SH2 domain specific anti Shoca-1 antibodies were generated and affinity-purified as described for antibody preparation C above except that a C-terminal cysteine was introduced as an anchoring residue.
Peptide Shoca#4: NHCOCH₃-DASGDFYSFLGVDPNRHC-CONH₂

Peptide#4 represents the 17 amino acid residues from position 376 through 392 of the human Shoca-1 protein sequence. This sequence stretch is identical in human and mouse Shoca-1. The peptide was coupled to a carrier protein as described above and injected into rabbits.

### Antibody preparation E

The human specific anti Shoca-2 antibodies were generated and affinity-purified as described for antibody preparation D.
Peptide Shoca#5: NHCOCH₃-QQMLADSINRMKC-CONH₂

Peptide#5 represents the 12 amino acid residues from position 181 through 192 of the human Shoca-2 protein sequence. This sequence has neither sequence identity with human Shoca-1 nor with mouse Shoca-1 or -2. The peptide was coupled to a carrier protein and injected into rabbits.

### Verification of antibody preparations

Western blot analysis was performed on various cell and protein material in order to verify the specificity of the antibody preparations. In particular, a blot was analysed with antibody preparation B. HEK293 cells transfected with various mouse Shoca-1 constructs namely 1. HEK293 with control plasmid; 2. HEK293 transfected with mouse Shoca-1 untagged; 3. HEK293 transfected with mouse Shoca-1 C-terminal HA-tag; and 4. HEK293 untransfected.

Antibody preparation B: Single bands corresponding to untagged and HA tagged Shoca-1 were seen in mouse Shoca-1 transfected HEK293 cells (data not shown).

Antibody preparation C: A single band is seen in the nuclear fraction of the human tumour cell line NCI-H520 at the correct molecular weight. No band was detected when staining mouse TEC cells verifying that this preparation is human specific.

Antibody preparation D: Nuclear detection of Shoca-1 in mouse TEC cells was carried out using a blot with antibody preparation D. Cytoplasmic and nuclear fractions of mouse thymic epithelial cells were used: 1. TEC1-2, cytoplasmic; 2. TEC1-2 nuclear (data not shown). Staining of recombinantly expressed mouse Shoca-1 SH2 domain. A blot with anti-penta His antibodies and antibody preparation D respectively were carried out. Bacterial expression of mouse Shoca-1 SH2 domain (14.5kDa including His tag) was identified, with higher levels being detected 5hr after induction in the supernatant: 1. supernatant collected 3hr after induction; 2. supernatant collected 5hr after induction; 3. pellet collected 5hr after induction (data not shown). Specificity for other SH2 domain proteins has been tested. GST fused Grb2-SH2 was not stained with antibody preparation D whereas the control anti-GST gave a nice band.

The western blot analysis were performed according to the following protocol. For total lysate, cells were suspended in lysis buffer (75 mM Tris pH8.0; 100 mM NaCl, 1% NP-40, 0.1 mM AEBSF and a protease inhibitor-mix) and centrifuged to remove debris. Supernatants were collected and same protein amounts were subjected to SDS-PAGE on 10% Bis/Tris gels under reducing conditions. Proteins were transferred onto PVDF membrane by semi-dry blotting (buffer: 25 mM Tris, 0.2M glycin, 20% ethanol). Membranes were blocked with 5% milk powder in TBST before incubation with affinity purified anti-Shoca antibodies (100-200 ng/ml). After 3 washes in TBST, membranes were incubated with HRP-coupled anti-rabbit-Ig antibodies (Amersham/Pharmacia) at a dilution of 1:2000. Finally, membranes were washed and developed by ECL-plus (Amersham/Pharmacia) chemiluminescence. For cytosolic versus nuclear lysates, cells were swollen for 15 min on ice in lysis buffer A (10 mM Hepes, 10 mM KCl, 0.1 mM EDTA, 1 mM EGTA, 1 mM DTT, protease inhibitor-mix). NP-40 was then added to a final concentration of 0.6% immediately followed by vortexing for 10 seconds and centrifugation for 30 seconds. The supernatant was referred to as cytosolic extract. The pellet was washed once in buffer A, resuspended in buffer B (20 mM Hepes, 0.4 M NaCl, 1 mM EDTA, 1 mM EGTA, 1 mM DTT, protease inhibitor-mix) and vortexed for 30 min at 4 °C. After centrifugation for 5 min the supernatant was recovered as nuclear extract.

### Example 3. Shoca-1 and Shoca-2 expression profile

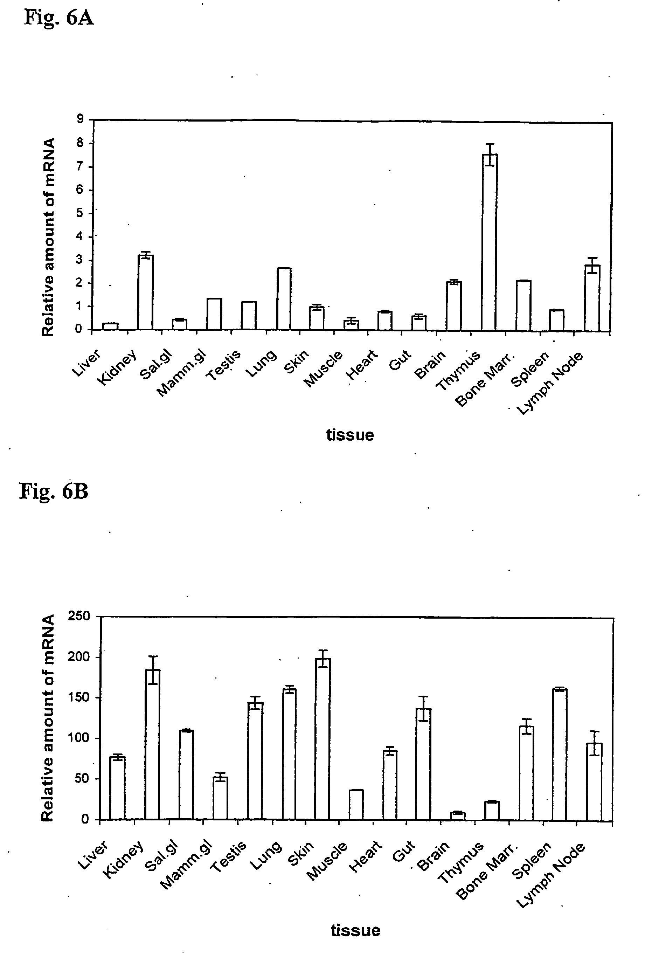

Shoca-1 is expressed in mice in thymic epithelial cells as early as the first detection of a thymic anlage and is also present in thymi of nude mice. Thus, Shoca-1 expression is independent of thymic lymphopoiesis and in particular the structured cross-talk between thymocytes and thymic epithelial cells. Shoca-1 expression has also been detected in bone marrow and in particular in stroma cells known to support hematopoiesis Brain, kidney, and lung constitute other sites where Shoca-1 is expressed at low abundancy when assayed by PCR (Figure 6), although Northern blotting was insufficiently sensitive to detect specific transcripts. By contrast, Shoca-2 expression is much more commonly expressed in different tissues (Figure 6).

### Example 4. Functional studies reveal epistatic placement of Shoca-1 in the Wnt/ β-catenin signalling pathway

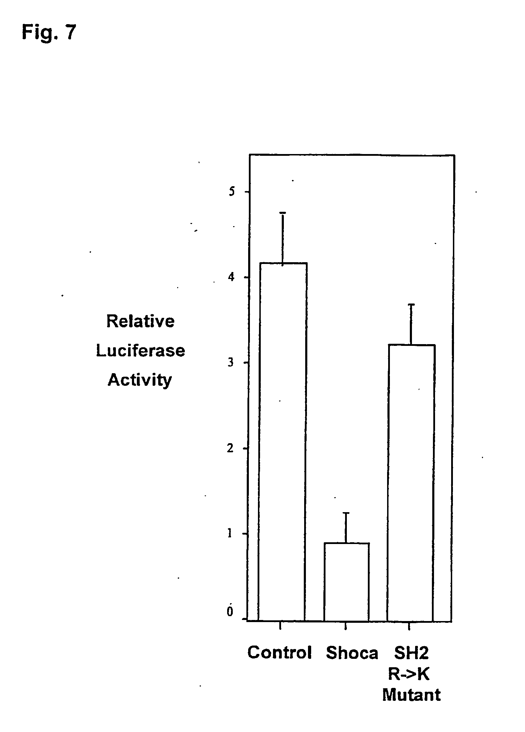

The functional in vitro analysis of Shoca-1 has revealed that its expression in thymic epithelial cells represses the transcriptional activation of β-catenin-LEF/TCF regulated activation of a reporter gene. This suppression is dependent on a functional SH2-domain as a point mutation in that domain ablates any repressive function (Figure 7). Arg350 (of mouse Shoca-1) in the SH2 domain was changed to a lysine residue. This arginine is located in the pTyr pocket and is critical for the SH2 domain function (Sawyer, 1998, Biopolymers 47: 243-261). It forms important contacts with the phosphate oxygen of the pTyr side chain of pTyr containing ligands to the SH2 domain. Overexpression of the R/K mutant of Shoca-1 has furthermore been associated with a decreased cellular proliferation. Electric mobility shift assays using Shoca-1 specific antibodies revealed that Shoca-1 is complexed with LEF/TCF.

This finding has been further confirmed in independent experiments where DNA sequences encoding the TCF-binding sequences could trap Shoca-1 from nuclear extracts. In these experiments, a biotinylated dsDNA adaptor containing a TCF binding site was incubated with nuclear extract from murine TEC 1-2 cells. The adaptor (and hence proteins complexed to the TCF binding site) was captured using paramagnetic streptavidin beads. The beads were washed, mixed with protein sample buffer, loaded on a PAGE gel and blotted onto a nitrocellulose membrane. Western analysis was then performed using polyclonal rabbit anti-Shoca antibody as a primary antibody.

The interaction with a transcriptionally active complex containing not only LEF/TCF but also β-catenin is further corroborated by confocal microscopy which shows that Shoca co-localizes with β-catenin in the nucleus. In addition, increased phosphorylation is observed of Shoca-1 after Li stimulation. This was demonstrated by incubating murine TEC 1-2 cells with medium containing 20mM lithium for different timepoints, immunoprecipitating Shoca-1 using polyclonal rabbit anti-Shoca antibody, separating the proteins on a PAGE gel and probing with a phosphotyrosine specific antibody in a Western analysis.

All in all, these data support a very central and epistatic placement of the Shoca proteins in the Wnt/β-catenin signalling pathway. In contrast to the observations in thymic epithelial cells, Shoca-1 overexpression in fibroblast stimulates LEF/TCF-dependent transcription of a reporter gene thus arguing for further tissue-specific molecules functionally and/or physically interacting with Shoca-1. Thus, Shoca-1 may effect a tissue specific repressor function in possible combination with a secondary molecule.

### Example 5. Tissue restricted expression and role of Shoca in oncogenesis.

The major role of Wnt signalling in adult tissues is the regulation of cell proliferation. Constitutive mutations in APC or β-catenin lead to hyperproliferation and carcinogenesis. Results from screening arrays containing normal and malignant tissue from different human organs has revealed that a number of tumours mainly of epithelial cell origin express Shoca-1 (Table 1).

**Table 1: Tissue micro array immunostaining for Shoca-1 expression**

| N = Number of samples that could be analysed | | | | | | | | |
|---|---|---|---|---|---|---|---|---|
| Score = Number of moderate to strong stainers divided by N | | | | | | | | |
| Percentage = value of score | | | | | | | | |
| Any score above 0.3 was taken as significant and marked with an asterisk | | | | | | | | |
| Pathology | N | Neg. | Weak | Mod. | Strong | Score | Percentage | |
| Adenomatoid tumor | 8 | 1 | 0 | 3 | 4 | 7/8 | 87.5% | * |
| Adrenal gland, adenoma | 15 | 12 | 0 | 3 | 0 | 3/15 | 20.0% | |
| Adrenal gland, cancer | 6 | 4 | 0 | 1 | 0 | 01/6 | 16.7% | |
| Angiosarcoma | 3 | 0 | 1 | 1 | 1 | 2/3 | 66.7% | * |
| Anus, squamous cell cancer | 5 | 3 | 0 | 2 | 0 | 2/6 | 40.0% | |
| Astrocytoma | 34 | 23 | 1 | 8 | 2 | 10/34 | 29.4% | |
| Benign histiocytoma | 26 | 5 | 5 | 6 | 10 | 16/25 | 61.5% | * |
| Breast, apocrine cancer | 2 | 0 | 1 | 1 | 0 | 1/2 | 50.0% | * |
| Breast, cribriform cancer | 8 | 5 | 1 | 2 | 0 | 2/8 | 25.0% | |
| Breast, ductal cancer | 46 | 23 | 8 | 12 | 3 | 15/46 | 32.6% | * |
| Breast, lobular cancer | 37 | 5 | 10 | 13 | 9 | 22/37 | 59.5% | * |
| Breast, medullary cancer | 27 | 19 | 4 | 4 | 0 | 4/27 | 14.8% | |
| Breast, mucinous cancer | 23 | 2 | 5 | 9 | 7 | 16/23 | 69.6% | * |
| Breast, Phylloides tumor | 11 | 3 | 0 | 4 | 4 | 8/4 | 72.7% | * |
| Breast, tubular cancer | 24 | 4 | 2 | 8 | 10 | 18/24 | 75.0% | * |
| Carcinoid tumor | 33 | 29 | 2 | 2 | 0 | 2/33 | 6.1% | |
| CML | 5 | 4 | 0 | 1 | 0 | 1/5 | 20.0% | |
| Colon adenoma, mild dysplasia | 45 | 5 | 8 | 18 | 4 | 32/45 | 48.9% | * |
| Colon adenoma, moderate dysplasia | 45 | 7 | 8 | 25 | 5 | 30/45 | 66.7% | * |
| Colon adenoma, severe dysplasia | 44 | 17 | 5 | 22 | 0 | 22/44 | 50.0% | * |
| Colon, adenocarcinoma | 45 | 33 | 5 | 7 | 0 | 7/45 | 15.6% | |
| Craniophayryngeoma | 4 | 0 | 1 | 2 | 1 | 3/4 | 75.0% | * |
| Dermatofibroma protuberans | 4 | 0 | 0 | 4 | 0 | 4/4 | 100.0% | * |
| Endometrioid stroma sarcoma | 4 | 0 | 1 | 0 | 0 | 0/4 | 0.0% | |
| Endometrium endometroid carcinoma | 47 | 35 | 6 | 4 | 2 | 6/47 | 12.8% | |
| Endometrium, serous carcinoma | 19 | 9 | 3 | 6 | 1 | 9/19 | 36.8% | * |
| Ependymoma | 7 | 0 | 1 | 4 | 2 | 6/7 | 85.7% | * |
| Epitheloid Hemangioma | 1 | | | | | 1/1 | 0.0% | |
| Epitheloid sarcoma | 2 | | | | | 2/2 | 0.0% | |
| Esophagus, adenocarcinoma | 6 | 3 | 2 | 1 | 0 | 1/6 | 16.7% | |
| Esophagus, small cell carcinoma | | | | | | | | |
| Esophagus, squamous cell carcinoma | 34 | 24 | 3 | 7 | 0 | 7/34 | 20.6% | |
| Esthesioneuroblastoma | 3 | 0 | 0 | 2 | 0 | 2/3 | 66.7% | * |
| Fibrosarcoma | 8 | 3 | 1 | 4 | 0 | 4/8 | 50.0% | * |
| Gall bladder, adenocarcinoma | 27 | 11 | 6 | 6 | 8 | 10/27 | 37.0% | * |
| Ganglioneuroma | 7 | 0 | 3 | 3 | 1 | 4/7 | 57.1% | * |
| GIST | 13 | 6 | 0 | 7 | 0 | 7/6 | 53.8% | * |
| Glioblastoma multiforme | 45 | 24 | 6 | 8 | 7 | 15/45 | 33.3% | * |
| Glomus tumor | 9 | 3 | 1 | 2 | 3 | 5/9 | 55.6% | * |
| Granular cell tumor | 8 | 2 | 4 | 2 | 0 | 2/8 | 25.0% | |
| Hemangiopericytoma | 16 | 4 | 2 | 3 | 3 | 6/16 | 37.5% | * |
| Hepatocellular carcinoma | 46 | 40 | 3 | 3 | 0 | 3/46 | 6.5% | |
| Hodgkin lymphoma, mixed cell | 15 | 8 | 3 | 4 | 0 | 4/15 | 26.7% | |
| Hodgkin lymphoma, nodular sclerosis | 9 | 6 | 3 | 0 | 0 | 0/9 | 0.0% | |
| Kapillary hemangioma | 26 | 2 | 3 | 3 | 18 | 21/26 | 80 .8% | * |
| Kaposi Sarcoma | 27 | 12 | 2 | 11 | 2 | 13/27 | 48.1% | * |
| Kidney, chromophobic cancer | 14 | 12 | 0 | 2 | 0 | 2/14 | 14.3% | |
| Kidney, clear cell cancer | 44 | 35 | 0 | 6 | 3 | 9/44 | 20.5% | |
| Kidney, oncocytoma | 10 | 2 | 0 | 7 | 1 | 8/10 | 80.0% | * |
| Kidney, papillary cancer | 43 | 29 | 6 | 3 | 5 | 8/43 | 18.6% | |
| Larynx, squamous cell carcinoma | 40 | 7 | 5 | 20 | 8 | 28/40 | 70.0% | * |
| Leiomyoblastoma | 7 | 4 | 0 | 3 | 3 | 6/7 | 85.7% | * |
| Leiomyoma | 55 | 16 | 11 | 18 | 10 | 28/55 | 50.9% | |
| Leiomyosarcoma | 46 | 29 | 6 | 9 | 2 | 11/46 | 23.9% | |
| Lipoma | 16 | 5 | 1 | 5 | 5 | 10/16 | 62.5% | |
| Liposarcoma | 23 | 7 | 8 | 4 | 4 | 8/23 | 34.8% | * |
| Lung, adenocarcinoma | 44 | 21 | 11 | 9 | 3 | 12/44 | 27.3% | |
| Lung, large cell cancer | 44 | 35 | 6 | 3 | 0 | 3/44 | 6.8% | |
| Lung, small cell cancer | 43 | 37 | 3 | 3 | 0 | 3/43 | 7.0% | |
| Lung, squamous cell carcinoma | 48 | 35 | 7 | 3 | 3 | 6/48 | 12.5% | |
| Malignant fibrous histiocytoma | 26 | 13 | 4 | 7 | 2 | 9/26 | 34.6% | * |
| Malignant mesothelioma | 25 | 14 | 6 | 2 | 3 | 5/25 | 20.0% | |
| Malignant Schwanoma | 12 | 3 | 0 | 9 | 0 | 9/12 | 75.0% | * |
| MALT lymphoma | 46 | 35 | 8 | 1 | 0 | 1/46 | 2.2% | |
| Medulloblastoma | 4 | 3 | 0 | 1 | 0 | 1/4 | 25.0% | |
| Meningeoma | 44 | 31 | 4 | 6 | 3 | 9/44 | 20.5% | |
| Neurofibroma | 36 | 17 | 9 | 10 | 0 | 10/36 | 27.8% | |
| NHL, diffuse large B | 22 | 17 | 3 | 1 | 1 | 2/22 | 9.1% | |
| NHL, others | 28 | 19 | 6 | 9 | 0 | 9/28 | 32.1% | * |
| Oligodendroglioma | 21 | 13 | 2 | 5 | 1 | 6/21 | 28.6% | |
| oral cavity, squamous cell carcinoma | 49 | 18 | 13 | 18 | 0 | 18/49 | 36.7% | * |
| Ovarian cancer, other types | 12 | 4 | 1 | 7 | 0 | 7/12 | 58.3% | * |
| Ovary, Brenner tumor | 8 | 5 | 0 | 3 | 0 | 3/8 | 37.5% | * |
| Ovary, endometroid cancer | 31 | 14 | 6 | 9 | 2 | 11/31 | 35.5% | * |
| Ovary, mucinous cancer | 14 | 4 | 0 | 3 | 7 | 10/14 | 71.4% | * |
| Ovary, serous cancer | 43 | 17 | 6 | 15 | 5 | 20/43 | 46.5% | * |
| Pancreas, adenocarcinoma | 43 | 28 | 4 | 8 | 3 | 11/43 | 25.6% | |
| Paraganglioma | 10 | 10 | 0 | 0 | 0 | 0/6 | 0.0% | |
| Parathyroid, adenoma | 26 | 9 | 0 | 17 | 0 | 17/26 | 65.4% | * |
| Parathyroid, cancer | 2 | 0 | 0 | 2 | 0 | 0 2/2 | 100.0% | * |
| Penile ca | 39 | 24 | 8 | 7 | 0 | 7/39 | 17.9% | |
| Pharynx, lamphoepithelial cancinoma | 5 | 2 | 3 | 0 | 0 | 0/5 | 0.0% | |
| Pheochromocytoma | 28 | 28 | 0 | 0 | 0 | 0/28 | 0.0% | |
| PNET | 15 | 11 | 1 | 3 | 0 | 3/15 | 20.0% | |
| Prostate cancer, hormon-refractory | 46 | 35 | 5 | 6 | 0 | 6/46 | 13.0% | |
| Prostate cancer, untreated | 47 | 26 | 12 | 8 | 1 | 9/47 | 19.1% | |
| Rhabdomyosarcoma | 13 | 6 | 1 | 4 | 2 | 6/13 | 46.2% | * |
| Salivary gland, acinus cell cancer | 5 | 4 | 0 | 1 | 0 | 1/5 | 20.0% | |
| Salivary gland, adenolymphoma | 26 | 3 | 4 | 17 | 2 | 19/26 | 73.1% | * |
| Salivary gland, cylindroma | 44 | 9 | 13 | 17 | 5 | 22/44 | 50.0% | * |
| Salivary gland, mucoepidermoid | 2 | 1 | 1 | 0 | 0 | 0/2 | 0.0% | |
| Salivary gland, Pleomorphic adenoma | 43 | 3 | 7 | 11 | 22 | 33/43 | 76.7% | * |
| Schwannoma | 39 | 31 | 3 | 4 | 1 | 5/39 | 12.8% | |
| Skin, basalioma | 41 | 13 | 9 | 19 | 0 | 19/41 | 46.3% | * |
| Skin, benign appendix tumor | 26 | 5 | 5 | 14 | 2 | 16/26 | 61.5% | * |
| Skin, benign nevus | 45 | 16 | 7 | 20 | 2 | 22/45 | 48.9% | * |
| Skin, malignant melanoma | 44 | 26 | 4 | 12 | 2 | 14/44 | 31.8% | |
| Skin, Merkel cell cancer | 4 | 2 | 0 | 2 | 0 | 2/4 | 50.0% | * |
| Skin, squamouse cell cancer | 39 | 10 | 9 | 18 | 2 | 20/39 | 51.3% | * |
| Small intestine, ademocarcinoma | 9 | 3 | 1 | 4 | 1 | 5/9 | 55:6% | * |
| Stomach, diffuse adenocarcinoma | 19 | 10 | 5 | 3 | 1 | 4/19 | 21.1% | |
| Stomach, intestinal adenocarcinoma | 41 | 26 | 5 | 10 | 0 | 10/41 | 24.4% | |
| Synovial sarcoma | 4 | 2 | 0 | 2 | 0 | 2/2 | 50.0% | * |
| Tendon sheet, giant cell tumor | 32 | 5 | 13 | 13 | 1 | 14/32 | 43.8% | * |
| Teratoma | 2 | 1 | 1 | 0 | 0 | 0/2 | 0.0% | |
| Testis, non-seminomatous cancer | 45 | 19 | 13 | 7 | 6 | 13/45 | 28.9% | |
| Testis, seminoma | 47 | 34 | 6 | 4 | 3 | 7/47 | 14.9% | |
| Thymoma | 23 | 17 | 1 | 5 | 0 | 5/23 | 21.7% | |
| Thyroid, adenoma | 43 | 17 | 15 | 10 | 1 | 11/43 | 25.6% | |
| Thyroid, anaplastic cancer | 7 | 1 | 0 | 2 | 4 | 6/7 | 85.7% | * |
| Thyroid, follicular cancer | 46 | 14 | 5 | 18 | 9 | 27/46 | 58.7% | * |
| Thyroid, medullary cancer | 8 | 4 | 1 | 0 | 3 | 3/8 | 37.5% | * |
| Thyroid, papillary cancer | 33 | 9 | 2 | 10 | 12 | 22/33 | 66.7% | * |
| Urinary bladder cancer, TCC invasive | 41 | 26 | 10 | 5 | 0 | 5/41 | 12.2% | |
| Urinary bladder cancer, TCC non-invasive | 40 | 22 | 8 | 8 | 2 | 10/40 | 25.0% | |
| Urinary bladder, adenocarcinoma | 4 | 1 | 1 | 2 | 0 | 2/4 | 50.0% | * |
| Urinary bladder, inverted papilloma | 1 | 0 | 0 | 1 | 0 | 1/1 | 100.0% | * |
| Urinary bladder, sarcomatoid cancer | 6 | 2 | 2 | 2 | 0 | 2/6 | 33.3% | |
| Urinary bladder, small cell cancer | 5 | 1 | 1 | 1 | 2 | 3/5 | 60.0% | * |
| Urinary bladder, squamous cell cancer | 7 | 2 | 1 | 1 | 3 | 4/7 | 57.1% | * |
| Urinary bladder, squamous cell carcinoma | 41 | 22 | 7 | 9 | 3 | 12/41 | 29.3% | |
| Uterus, carcinsarcoma | 6 | 2 | 2 | 1 | 1 | 2/6 | 33.3% | * |
| Uterus, cervix, adenocarcinoma | 2 | 0 | 0 | 2 | 0 | 2/2 | 100.0% | * |
| Uterus, cervix, CIN III | 16 | 0 | 3 | 13 | 0 | 13/16 | 81.3% | * |
| Vagina, squamous, cell cancer | 5 | 3 | 2 | 0 | 0 | 0/5 | 0.0% | |
| Vulva, squamous, cell cancer | 39 | 14 | 11 | 13 | 1 | 14/39 | 35.9% | * |

A set of tissue micro arrays (7 slides each) was analysed for Shoca expression. The set contained about 3000 tumours of 129 different tumour categories. Immunostaining was first optimised on test slides. The conditions described below were considered optimal. Then, all TMA slides used for this study were immunostained in one experiment. Only the negative control reaction was performed later (when reagents were provided). Immunostainings were done according to the following protocols:
Antibody: Shoca-1 specific type B
Slide pre-treatment: pronase, 15 min, 37° C.
Peroxidase blocking: 0.3% H₂O₂/Methanol, 30 minutes
Antibody dilution: 1:32'000
Incubation: overnight (4° C)
Detection system: ABC; DAB
Positive control: thymus epithelial cells
Negative control: serum of the animal producing the antibody (after antibody extraction)

Using the conditions described above, the negative control reactions were completely negative while a staining of various intensities was observed for the Shoca-1 antibody in many tumours and normal tissues. All sections (stained and control sections) were reviewed by one pathologist on one day to maximise the internal consistency. For all tumour samples the fraction of positive cells were estimated and staining intensity was recorded according to a four step scale (0 - 3+). To categorise the tumours an arbitrarily selected system was used (Table 2).

**Table 2: Categorisation of tumours**

| **Category** | **Definition** |
|---|---|
| Negative | no staining |
| Weak | 0-50% of cells 1+ |
| Moderate | >50% 1+ or <50% 2+ |
| Strong | >50% 2+ or any 3+ staining |

The protein seems to be expressed in many different cell types and neoplastic tissues. It is possible that the protein is also expressed (at lower level) in tissues that were considered negative at the selected immunohistochemistry conditions. Despite all inherent shortcomings of immunohistochemistry, it can be expected that the different staining levels as determined on the tissue microarrays do at least represent true differences in the expression level of Shoca-1. This means, that tumours that are scored as 3+ will in general have a higher expression level than 2+ tumours, 2+ tumours will have a higher expression level than 1+ tumours, and 1+ tumours will express more Shoca-1 than tumours that are scored negative.

These data demonstrate the involvement of Shoca-1 in distinct tumour types including breast cancer, colon cancer and cervical cancer. A comprehensive analysis of the different tissues and tumour entities indicated further that normal tissue from a broad range of organs was low positive to negative for Shoca-1 while expression of this molecule was associated with less malignant tumour forms in these tissues. Moreover, cancerous progression coincided with a loss of Shoca-1 expression in many of the tumours (Table 3). This finding is particularly interesting as there is presently to our knowledge extremely few clinically relevant prognostic markers are available which are down-modulated or lost upon tumour progression in breast, colon and cervical cancer.

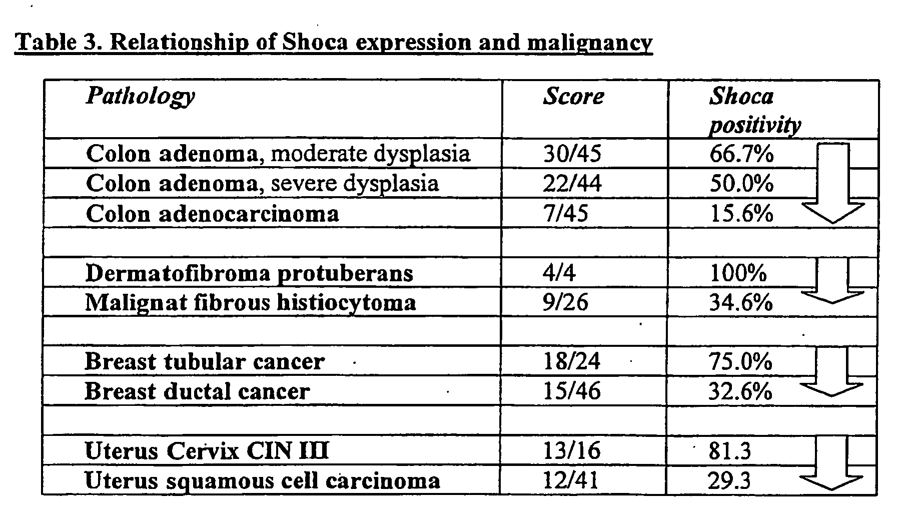

### Example 6. Shoca gene expression in Human malignancies

The role of Shoca-1 and Shoca-2 in malignancy was examined by a reverse transcription polymerase chain reaction (RT-PCR) in over 50 human cancer cell lines, representing various malignancies and different stages of cancer development.

The starting material was total RNA isolated from cultured cell lines by Qiagen Rneasy Total RNA isolation kit. Starting with 1µg total RNA, random hexamers (Roche) were used to prime a reverse transcription reaction (Titan One Tube RT-PCR (Roche). cDNA was amplified in two rounds for both Shoca-1 and Shoca-2. In both primary PCR reactions, a full length amplicon was generated, and a portion of this was amplified in the secondary PCR reaction. The primers used are specified below:

**Table 4: PCR primers**

| Primary PCR Primers for Shoca-1. | | | |
|---|---|---|---|
| Name | Nucleotide Position | | Sequence (5'-3') |
| Shoca1 5' | | | TGC TGC AGC AGA TCC TGC AC |
| Shoca1 3' | | | CCT GAA GTA ACT CTC CTC C |
| | | | |
| Shoca_F1 | n258-278 | | CCCTGGTGACAAGCCCTACGA |
| Shoca_R1 | n710-731 | | ATAGCACGGAGCGAGTGGTGTC |
| | | | |

| Primary PCR Primers for Shoca-2 | | | |
|---|---|---|---|
| Shoca2_F2 | 348-365 | | TCA CTC TGA AGA ATT CAC |
| Shoca2_R2 | 1063-1046 | | TGA GTG TGA GAA TTC CAT |
| | | | |

| Nested PCR primers for Shoca-2 Reactions | | | |
|---|---|---|---|
| Shoca2_F3 | 511-528 | | GAT CAC TCT CCA GTT CTT |
| Shoca2_R3 CTG | 717-697 | | GGA TTT TCG CAG AGA TGC |

PCR products were separated by electrophoresis on a 0.8% agarose gel overnight and examined under UV light after staining with 0.5 µg/ml ethidium bromide. The table below details the findings. Results are given as Pos, where a band of the correct molecular weight was detected, and Neg, where no PCR product was detected.

**Table 5: Shoca gene expression in various human cancer cells lines**

| | | | | |
|---|---|---|---|---|
| Results for 20 of the 54 cell lines are listed. All the 34 remaining cell lines were negative for Shoca-1 and positive for Shoca-2. | | | | |

| Name | | Tumour Type | Shoca-1 | Shoca-2 |
|---|---|---|---|---|
| 1. | T47 D/6 | Breast | NEG | POS |
| **2.** | **PAN 02** | **Pancreatic** | **NEG** | **NEG** |
| 3. | U-87 | Glioblastoma | NEG | POS |
| 4. | T84 MCB | lung met from colon carcinoma | NEG | POS |
| **5.** | **NCI-H520** | **Squamous cell carcinoma lung** | **POS** | **POS** |
| **6.** | **KLE** | **Endometrial carcinoma** | **NEG** | **NEG** |
| **7.** | **SK-LU- 1** | **Lung adenocarcinoma** | **N.D.** | **POS** |
| **8.** | **U-251 MG** | **Glioma** | **NEG** | **NEG** |
| **9.** | **NCI-H128.** | **SCLC met pleural effusion** | **NEG** | **NEG** |
| 10. | SCATT | Renal | NEG | POS |
| 11. | NCI-H460 | Large cell lung carcinoma | NEG | POS |
| 12. | LoVo | Colon met site supraclavicular | NEG | POS |
| 13. | GLC-08 | SCLC | NEG | POS |
| 14. | Hep G2 | Hepatocellular carcinoma | NEG | POS |
| 15. | HT1376 | Urinary bladder carcinoma | NEG | POS |
| **16.** | **N417** | **Small Cell Lung** | **NEG** | **NEG** |
| 17. | LN Cap | Prostate | NEG | POS |
| **18.** | **U105MG** | **Glioma** | **NEG** | **NEG** |
| 19. | SW 837 | Rectal adenocarcinoma | NEG | POS |
| **20.** | **Colo 320** | **Colon** | **NEG** | **NEG** |
| 21. | no template | | NEG | NEG |

The findings for Shoca-1 demonstrate that it is rarely expressed in human cancer cell lines, with only one (NCI H520) clearly positive. Shoca-2 was far more widely expressed in human tumours than Shoca-1. Only 7 cell lines were clearly negative of the 54 tested. These were PAN02, KLE, U251MG, NCIH128, N417, U105MG, and Colo320. These tumours cover 6 different tumour types, with only Gliomas represented twice. Another Glioma (U87), was positive for Shoca-2.

## Claims

1. An isolated Shoca polypeptide comprising:
(i) the amino acid sequence of SEQ ID NO: 2 or SEQ ID NO: 4; or
(ii) a variant thereof having at least 70% identity to the amino acid sequence of SEQ ID NO: 2 or SEQ ID NO: 4 which is capable of interacting with a polypeptide of the wnt signalling pathway; or
(iii) a fragment of (i) of at least 20 amino acids in length which is capable of interacting with a polypeptide of the wnt signalling pathway selected from β-catenin, T-cell factor (TCF) and leucocyte enhancer factor (LEF).

2. A polypeptide according to claim 1 wherein the variant (ii) has at least 85% identity to the amino acid sequence of SEQ ID NO: 2 or SEQ ID NO: 4.

3. A polypeptide according to any one of the preceding claims which comprises a functional SH2 domain.

4. A polypeptide according to any one of the preceding claims which comprises a coiled-coil domain.

5. A polypeptide according to any one of the preceding claims which comprises a nuclear localization sequence.

6. A polypeptide according to claim 5 which comprises an N-terminal domain.

7. A polynucleotide encoding a polypeptide according to any one of the preceding claims.

8. A polynucleotide encoding a Shoca polypeptide capable of interacting with a polypeptide of the wnt signalling pathway, which polynucleotide comprises:
(i) the nucleic acid sequence of SEQ ID NO: 1 or SEQ ID NO: 3 and/or a sequence complementary thereto;
(ii) a sequence that is degenerate as a result of the genetic code to a sequence as defined in (i); or
(iii) a sequence having at least 85% identity to a sequence as defined in (i) or (ii), over the full length of said sequence of (i) or (ii).

9. A polynucleotide according to claim 7 or 8 which is cDNA or mRNA.

10. An expression vector comprising a polynucleotide according to any one of claims 7 to 9.

11. A host cell comprising a vector according to claim 10.

12. An antibody specific for a polypeptide according to any one of claims 1 to 6.

13. An antibody according to claim 12 which is specific for the amino acid sequence of SEQ ID NO: 19, 20 or 21.

14. A method of identifying an agent capable of modulating the wnt signalling pathway, which method comprises:
(i) providing:
- a polypeptide comprising the amino acid sequence of SEQ ID NO: 2, 4, 6 or 8 or a variant thereof having at least 70% identity to the sequence of SEQ ID NO: 2, 4, 6 or 8, or a fragment of any thereof of at least 20 amino acids which variant or fragment is capable of interacting with a polypeptide of the wnt signalling pathway selected from β-catenin, TCF and LEF; or
- a polynucleotide encoding said polypeptide; and
- a test agent;
(ii) contacting the polypeptide or polynucleotide and the test agent; and
(iii) determining whether the test agent has any effect on the expression of said polypeptide or on a function or property of said polypeptide thereby determining whether the test agent is capable of modulating Shoca activity.

15. A method according to claim 14, wherein the agent is capable of inhibiting Shoca activity.

16. A method according to claim 14, wherein said polynucleotide is mRNA.

17. A method according to claim 14 or 15, wherein step (iii) comprises monitoring cell proliferation, differentiation, growth or survival.

18. A method according to claim 14 or 15, wherein step (iii) comprises monitoring the intracellular location of Shoca.

19. A method according to claim 14 or 15, wherein step (ii) comprises contacting a polynucleotide encoding said polypeptide with a test agent and step (iii) comprises monitoring the expression of said polypeptide.

20. A method according to claim 19, wherein said polynucleotide is DNA.

21. A method according to claim 14 or 15 wherein step (i) further comprises providing a molecule of the wnt signalling pathway capable of interacting with Shoca, step (ii) comprises contacting the polypeptide, test agent and wnt signalling molecule under conditions suitable for the interaction of the polypeptide and wnt signalling molecule and step (iii) comprises monitoring the interaction of the polypeptide and wnt signalling molecule.

22. A method according to claim 21, wherein the wnt signalling molecule is a nuclear molecule.

23. A method according to claim 22, wherein the nuclear molecule is β-catenin, T-cell factor (TCF), leucocyte enhancer factor (LEF) or any combination of β-catenin and TCF and/or LEF.

24. A method according to claim 14 or 15 wherein step (i) further comprises providing a reporter construct wherein the reporter gene is under the transcriptional control of LEF and/or TCF, step (ii) comprises contacting the polypeptide or polynucleotide, test agent and reporter construct under conditions suitable for expression of the reporter gene; and step (iii) comprises monitoring expression of the reporter gene.

25. A method for identifying a tissue specific modulator of Shoca activity, which method comprises a method according to any one of claims 14 to 24 wherein said test agent is a cellular component from a cell of said tissue, or wherein said method is carried out in a cell from said tissue.

26. A method of diagnosing cancer which method comprises determining the level of Shoca expression in a tissue sample from a subject.

27. A method of predicting the progression of a tumour which method comprises determining the level of Shoca expression in a tissue sample from a subject.

28. A method according to claim 26 or 27 wherein the amount of Shoca protein or mRNA is determined.

29. A method according to any one of claims 26 to 28 wherein the tissue sample is a biopsy from a tissue selected from colon, breast or cervix.

30. A method according to any one of claims 26 to 29 which method comprises contacting said sample with an agent that interacts with Shoca protein or mRNA and monitoring the binding of the agent to the protein or mRNA.

31. A method according to claim 30 wherein the agent is an antibody.

32. A method according to claim 30 wherein the agent is identifiable by a method according to any one of claims 14 to 25.

## Patentansprüche

1. Isoliertes Shoca-Polypeptid, umfassend:
(i) die Aminosäuresequenz der SEQ ID Nr. 2 oder SEQ ID Nr. 4; oder
(ii) eine Variante davon mit wenigstens 70 % Identität zu der Aminosäuresequenz der SEQ ID Nr. 2 oder SEQ ID Nr. 4, welche zum Interagieren mit einem Polypeptid des Wnt-Signalwegs fähig ist; oder
(iii) ein Fragment von (i) von wenigstens 20 Aminosäuren Länge, welches zum Interagieren mit einem Polypeptid des Wnt-Signalwegs fähig ist, ausgewählt aus β-Catenin, T-Zell-Faktor (TCF) und Leukozyt-Enhancer-Faktor (LEF).

2. Polypeptid nach Anspruch 1, wobei die Variante (ii) wenigstens 85 % Identität zu der Aminosäuresequenz der SEQ ID Nr. 2 oder SEQ ID Nr. 4 aufweist.

3. Polypeptid nach einem der vorangehenden Ansprüche, welches eine funktionelle SH2-Domäne umfasst.

4. Polypeptid nach einem der vorangehenden Ansprüche, welches eine Coiled-Coil-Domäne umfasst.

5. Polypeptid nach einem der vorangehenden Ansprüche, welches eine nukleäre Lokalisationssequenz umfasst.

6. Polypeptid nach Anspruch 5, welches eine N-terminale Domäne umfasst.

7. Polynukleotid, das ein Polypeptid nach einem der vorangehenden Ansprüche codiert.

8. Polynukleotid, das ein Shoca-Polypeptid codiert, welches zum Interagieren mit einem Polypeptid des Wnt-Signalwegs fähig ist, welches Polynukleotid umfasst:
(i) die Nukleinsäuresequenz der SEQ ID Nr. 1 oder SEQ ID Nr. 3 und/oder eine dazu komplementäre Sequenz;
(ii) eine Sequenz, die als Folge des genetischen Codes zu einer Sequenz, wie in (i) definiert, degeneriert ist; oder
(iii) eine Sequenz mit wenigstens 85 % Identität zu einer Sequenz, wie in (i) oder (ii) definiert, über die volle Länge dieser Sequenz von (i) oder (ii).

9. Polynukleotid nach Anspruch 7 oder 8, welches cDNA oder mRNA ist.

10. Expressionsvektor, umfassend ein Polynukleotid nach einem der Ansprüche 7 bis 9.

11. Wirtszelle, umfassend einen Vektor nach Anspruch 10.

12. Antikörper, der spezifisch für ein Polypeptid nach einem der Ansprüche 1 bis 6 ist.

13. Antikörper nach Anspruch 12, welcher für die Aminosäuresequenz der SEQ ID Nr. 19, 20 oder 21 spezifisch ist.

14. Verfahren zum Identifizieren eines Agens, das zum Modulieren des Wnt-Signalwegs fähig ist, welches Verfahren umfasst:
(i) Bereitstellen:
- eines Polypeptids, umfassend die Aminosäuresequenz der SEQ ID Nr. 2, 4, 6 oder 8 oder eine Variante davon mit wenigstens 70 % Identität zu der Sequenz der SEQ ID Nr. 2, 4, 6 oder 8, oder ein Fragment von irgendeinem davon aus wenigstens 20 Aminosäuren, welche Variante oder Fragment zum Interagieren mit einem Polypeptid des Wnt-Signalwegs fähig ist, ausgewählt aus β-Catenin, TCF und LEF; oder
- ein Polynukleotid, das dieses Polypeptid codiert; und
- ein Testagens;
(ii) Kontaktieren des Polypeptids oder Polynukleotids und des Testagens; und
(iii) Bestimmen, ob das Testagens irgendeine Wirkung auf die Expression des Polypeptids oder auf eine Funktion oder Eigenschaft des Polypeptids aufweist, dabei Bestimmen, ob das Testagens zur Modulierung der Shoca-Aktivität fähig ist.

15. Verfahren nach Anspruch 14, wobei das Agens zur Hemmung der Shoca-Aktivität fähig ist.

16. Verfahren nach Anspruch 14, wobei das Polynukleotid mRNA ist.

17. Verfahren nach Anspruch 14 oder 15, wobei Schritt (iii) das Überwachen der Zellproliferation, -Differenzierung, -Wachstum oder -Überleben umfasst.

18. Verfahren nach Anspruch 14 oder 15, wobei Schritt (iii) das Überwachen der intrazellulären Lokalisation des Shoca umfasst.

19. Verfahren nach Anspruch 14 oder 15, wobei Schritt (ii) das Kontaktieren eines Polynukleotids, das dieses Polypeptid codiert, mit einem Testagens umfasst und Schritt (iii) das Überwachen der Expression des Polypeptids umfasst.

20. Verfahren nach Anspruch 19, wobei das Polynukleotid DNA ist.

21. Verfahren nach Anspruch 14 oder 15, wobei Schritt (i) außerdem das Bereitstellen eines Moleküls des Wnt-Signalwegs umfasst, welches zum Interagieren mit Shoca fähig ist, Schritt (ii) das Kontaktieren des Polypeptids, Testagens und Wnt-Signalmoleküls unter Bedingungen umfasst, die für die Interaktion des Polypeptids und Wnt-Signalmoleküls geeignet sind, und Schritt (iii) das Überwachen der Interaktion des Polypeptids und Wnt-Signalmoleküls umfasst.

22. Verfahren nach Anspruch 21, wobei das Wnt-Signalmolekül ein nukleäres Molekül ist.

23. Verfahren nach Anspruch 22, wobei das nukleäre Molekül *β*-Catenin, T-Zell-Faktor (TCF), Leukozyt-Enhancer-Faktor (LEF) oder irgendeine Kombination von β-Catenin und TCF und/oder LEF ist.

24. Verfahren nach Anspruch 14 oder 15, wobei Schritt (i) außerdem das Bereitstellen eines Reporterkonstrukts umfasst, wobei das Reportergen unter der Transkriptionskontrolle von LEF und/oder TCF steht; Schritt (ii) das Kontaktieren von Polypeptid oder Polynukleotid, Testagens und Reporterkonstrukt unter Bedingungen umfasst, die für die Expression des Reportergens geeignet sind; und Schritt (iii) das Überwachen der Expression des Reportergens umfasst.

25. Verfahren zur Identifizierung eines gewebespezifischen Modulators der Shoca-Aktivität, welches Verfahren ein Verfahren nach einem der Ansprüche 14 bis 24 umfasst, wobei das Testagens eine zelluläre Komponente von einer Zelle dieses Gewebes ist, oder wobei das Verfahren in einer Zelle aus diesem Gewebe vorgenommen wird.

26. Verfahren zum Diagnostizieren von Krebs, welches Verfahren das Bestimmen der Höhe der Shoca-Expression in einer Gewebeprobe von einem Patienten umfasst.

27. Verfahren zum Voraussagen der Weiterentwicklung eines Tumors, welches Verfahren das Bestimmen der Höhe der Shoca-Expression in einer Gewebeprobe von einem Patienten umfasst.

28. Verfahren nach Anspruch 26 oder 27, wobei die Menge an Shoca-Protein oder mRNA bestimmt wird.

29. Verfahren nach einem der-Ansprüche 26 bis 28, wobei die Gewebeprobe eine Biopsie von einem Gewebe, ausgewählt aus Dickdarm, Brust oder Hals, ist.

30. Verfahren nach einem der Ansprüche 26 bis 29, welches Verfahren das Kontaktieren der Probe mit einem Agens, das mit Shoca-Protein oder mRNA interagiert, und Überwachen der Bindung des Agens an das Protein oder mRNA umfasst.

31. Verfahren nach Anspruch 30, wobei das Agens ein Antikörper ist.

32. Verfahren nach Anspruch 30, wobei das Agens mittels eines Verfahrens nach einem der Ansprüche 14 bis 25 identifizierbar ist.

## Revendications

1. Polypeptide de Shoca isolé comprenant
(i) la séquence d'acides aminés de SEQ ID NO : 2 ou SEQ ID NO : 4 ;
ou
(ii) un variant de celle-ci ayant au moins 70 % d'identité avec la séquence d'acides aminés de SEQ ID NO : 2 ou SEQ ID NO : 4 qui est capable d'interagir avec un polypeptide de la voie de signalisation wnt ; ou
(iii) un fragment de (i) d'une longueur d'au moins 20 acides aminés qui est capable d'interagir avec un polypeptide de la voie de signalisation wnt choisi parmi la β-caténine, le facteur de lymphocyte T (TCF) et le facteur de stimulation des leucocytes (LEF).

2. Polypeptide selon la revendication 1 dans lequel le variant (ii) a au moins 85 % d'identité avec la séquence d'acides aminés de SEQ ID NO : 2 ou SEQ ID NO : 4.

3. Polypeptide selon l'une quelconque des revendications précédentes qui comprend un domaine SH2 fonctionnel.

4. Polypeptide selon l'une quelconque des revendications précédentes qui comprend un domaine coiled-coil.

5. Polypeptide selon l'une quelconque des revendications précédentes qui comprend une séquence de localisation nucléaire.

6. Polynucléotide selon la revendication 5 comprenant un domaine N-terminal.

7. Polynucléotide codant pour un polypeptide selon l'une quelconque des revendications précédentes.

8. Polynucléotide codant pour un polypeptide Shoca capable d'interagir avec un polypeptide de la voie de signalisation wnt, lequel polynucléotide comprend :
(i) la séquence d'acide nucléique de SEQ ID NO : 1 ou SEQ ID NO : 3 ;
et/ou une séquence complémentaire de celles-ci ;
(ii) une séquence qui est dégénérée en raison du code génétique en une séquence telle que définie en (i) ; ou
(iii) une séquence ayant au moins 85 % d'identité avec une séquence telle que définie en (i) ou (ii), sur la longueur complète de ladite séquence de (i) ou (ii).

9. Polynucléotide selon la revendication 7 ou 8 qui est un ADNc ou un ARNm.

10. Vecteur d'expression comprenant un polynucléotide selon l'une quelconque des revendications 7 à 9.

11. Cellule hôte comprenant un vecteur selon la revendication 10.

12. Anticorps spécifique d'un polypeptide selon l'une quelconque des revendications 1 à 6.

13. Anticorps selon la revendication 12 qui est spécifique de la séquence d'acides aminés de SEQ ID NO : 19, 20 ou 21.

14. Méthode d'identification d'un agent capable de moduler la voie de signalisation wnt, laquelle méthode comprend :
(i) la fourniture :
- d'un polypeptide comprenant la séquence d'acides aminés de SEQ ID NO :2, 4, 6 ou 8 ou un variant de celles-ci ayant au moins 70 % d'identité avec la séquence de SEQ ID NO : 2, 4, 6 ou 8, ou un fragment d'une quelconque de celles-ci d'au moins 20 acides aminés, lequel variant ou fragment est capable d'interagir avec un polypeptide de la voie de signalisation wnt choisi parmi la β-caténine, TCF et LEF ; ou
- un polynucléotide codant pour ledit polypeptide ; et
- un agent de test ;
(ii) la mise en contact du polypeptide ou du polynucléotide et de l'agent de test ; et
(iii) le fait de déterminer si l'agent de test a un effet quelconque sur l'expression dudit polypeptide ou sur une fonction ou propriété dudit polypeptide déterminant ainsi si l'agent de test est capable de moduler l'activité de Shoca.

15. Méthode selon la revendication 14, dans laquelle l'agent est capable d'inhiber l'activité de Shoca.

16. Méthode selon la revendication 14, dans laquelle ledit polynucléotide est un ARNm.

17. Méthode selon la revendication 14 ou 15, dans laquelle l'étape (iii) comprend le suivi de la prolifération, différenciation, croissance ou survie cellulaire.

18. Méthode selon la revendication 14 ou 15, dans laquelle l'étape (iii) comprend le suivi de la position intracellulaire de Shoca.

19. Méthode selon la revendication 14 ou 15, dans laquelle l'étape (ii) comprend la mise en contact d'un polynucléotide codant pour ledit polypeptide avec un agent de test et l'étape (iii) comprend le suivi de l'expression dudit polypeptide.

20. Méthode selon la revendication 19, dans laquelle ledit polynucléotide est un ADN.

21. Méthode selon la revendication 14 ou 15, dans laquelle l'étape (i) comprend en outre la fourniture d'une molécule de la voie de signalisation wnt capable d'interagir avec Shoca, l'étape (ii) comprend la mise en contact du polypeptide, de l'agent de test et de la molécule de signalisation wnt dans des conditions appropriées pour l'interaction du polypeptide et de la molécule de signalisation wnt et l'étape (iii) comprend le suivi de l'interaction du polypeptide et de la molécule de signalisation wnt.

22. Méthode selon la revendication 21, dans laquelle la molécule de signalisation wnt est une molécule nucléaire.

23. Méthode selon la revendication 22, dans laquelle la molécule nucléaire est la β-caténine, le facteur de lymphocytes T (TCF), le facteur de stimulation de leucocytes (LEF) ou une combinaison quelconque de β-caténine et de TCF et/ou LEF.

24. Méthode selon la revendication 14 ou 15 dans laquelle l'étape (i) comprend en outre la fourniture d'une construction rapporteur dans laquelle le gène rapporteur est sous le contrôle transcriptionnel de LEF et/ou TCF, l'étape (ii) comprend la mise en contact du polypeptide ou du polynucléotide, de l'agent de test et de la construction rapporteur dans des conditions appropriées pour l'expression du gène rapporteur ; et l'étape (iii) comprend le suivi de l'expression du gène rapporteur.

25. Méthode d'identification d'un modulateur tissu spécifique de l'activité Shoca, laquelle méthode comprend une méthode selon l'une quelconque des revendications 14 à 24 dans laquelle ledit agent de test est un composant cellulaire d'une cellule dudit tissu, ou dans laquelle ladite méthode est réalisée dans une cellule dudit tissu.

26. Méthode de diagnostic d'un cancer, laquelle méthode comprend la détermination du niveau d'expression de Shoca dans un échantillon de tissu d'un sujet.

27. Méthode de prédiction de la progression d'une tumeur, laquelle méthode comprend la détermination du niveau d'expression de Shoca dans un échantillon de tissu d'un sujet.

28. Méthode selon la revendication 26 ou 27 dans laquelle la quantité de protéine ou d'ARNm de Shoca est déterminée.

29. Méthode selon l'une quelconque des revendications 26 à 28 dans laquelle l'échantillon de tissu est une biopsie d'un tissu choisi parmi le côlon, le sein ou le col de l'utérus.

30. Méthode selon l'une quelconque des revendications 26 à 29, laquelle méthode comprend la mise en contact dudit échantillon avec un agent qui interagit avec la protéine ou l'ARNm de Shoca et le suivi de la liaison de l'agent avec la protéine ou l'ARNm.

31. Méthode selon la revendication 30 dans laquelle l'agent est un anticorps.

32. Méthode selon la revendication 30 dans laquelle l'agent est identifiable par une méthode selon l'une quelconque des revendications 14 à 25.
